# EUROPÄISCHE PATENTSCHRIFT

(11) **EP 2 953 671 B1**
(45) Veröffentlichungstag und Bekanntmachung des Hinweises auf die Patenterteilung: **18.10.2017**
(21) Anmeldenummer: 14703269.2
(22) Anmeldetag: 05.02.2014
(51) Int. Cl.: A61M 5/315, A61M 5/24

(54) **INJEKTIONSGERÄT**
INJECTION DEVICE
DISPOSITIF D'INJECTION

(30) Priorität: 08.02.2013 DE 202013001350 U
(43) Veröffentlichungstag der Anmeldung: 16.12.2015
(60) Teilanmeldung: 17001491.4
(73) Patentinhaber: Haselmeier AG, 9001 St. Gallen (CH)
(72) Erfinder: KEITEL, Joachim, 73728 Esslingen (DE); MACDONALD, Daniel, Brossard, Québec J4Z 0J4 (CA); BECHTOLD, Herbert, 78588 Denkingen (DE)
(74) Vertreter: Reinhardt, Annette
(86) Internationale Anmeldenummer: PCT/EP2014/000313
(87) Internationale Veröffentlichungsnummer: WO 2014/121929

(56) Entgegenhaltungen:
- WO-A1-2006/076921
- WO-A1-2010/140974
- WO-A1-2011/101349

## Beschreibung

Die Erfindung betrifft ein Injektionsgerät der im Oberbegriff des Anspruchs 1 angegebenen Gattung.

Aus der EP 1 610 848 B2 ist ein Injektionsgerät bekannt, bei dem zum Einstellen einer Injektionsdosis ein Dosierorgan gedreht wird, bis die gewünschte Dosis in einem Sichtfenster erscheint. Aufgrund der Drehung bewegt sich das Dosierorgan in distaler Richtung des Injektionsgeräts, entfernt sich also von einer auf das Injektionsgerät aufgesetzten Injektionsnadel. Das Injektionsgerät besitzt eine Rasteinrichtung, die eine Vielzahl von Raststellungen besitzt. Der Bediener kann die Dosis dadurch auch durch Abzählen einer Anzahl von spürbaren und hörbaren Raststellungen einstellen. Ein Einstellen von Zwischenstellungen zwischen zwei Raststellungen ist nicht möglich. Das Injektionsgerät kann eine Torsionsfeder besitzen, die beim Einstellen einer Dosis funktionslos ist. Beim Auspressen einer eingestellten Dosis aus dem Behälter unterstützt die Torsionsfeder die Verdrehung des Einstellglieds und damit die Injektion.

Bei dem in der EP 1 610 848 B2 gezeigten Injektionsgerät können unterschiedlichste Mengen an Injektionsflüssigkeit eingestellt werden. Die möglichen einzustellenden Mengen werden über die Rasteinrichtung vorgegeben. Von Positionen des Bedienelements, die vom Hersteller nicht vorgesehenen Mengen an Injektionsflüssigkeit entsprechen, springt das Bedienelement in die nächstliegende vorgesehene Position.

Damit der Bedienknopf aus einer Zwischenposition selbsttätig und sicher in eine Rastposition springt, muss die Raste hinreichend stark sein, und die radialen Rastpositionen müssen hinreichend nahe beieinander liegen. Die Stärke der Rastung beeinflusst jedoch das Drehmoment, das der Anwender aufwenden muss, um den Bedienknopf zu drehen und die Dosis einzustellen. Der konstruktiv mögliche Abstand der Rastpositionen ist dadurch weitgehend vorgegeben und kann nur in engen Grenzen auf den Anwendungsfall angepasst werden.

Der Erfindung liegt die Aufgabe zugrunde, ein Injektionsgerät der gattungsgemäßen Art zu schaffen, bei dem nur vorgegebene Mengen von Injektionsflüssigkeit aus dem Behälter ausgepresst werden können und das eine gute Anpassung auf den gewünschten Anwendungsfall erlaubt.

Diese Aufgabe wird durch ein Injektionsgerät mit den Merkmalen des Anspruchs 1 gelöst.

Das Dosierorgan besitzt mindestens eine Injektionsstellung, in der eine vom Hersteller vorgesehene Dosis an Injektionsflüssigkeit eingestellt ist. Dabei kann nur eine einzige Injektionsstellung vorgesehen sein, wenn ein Medikament zu verabreichen ist, das nur in einer vorgegebenen Dosis verabreicht werden soll. Es können jedoch auch mehrere Injektionsstellungen vorgesehen sein, beispielsweise, wenn ein Medikament abhängig von Indikation und Bediener in mehreren unterschiedlichen vorgegebenen Dosen verabreicht werden kann. Das Injektionsgerät besitzt eine Rasteinrichtung, wobei jeder Injektionsstellung des Dosierorgans eine Raststellung der Rasteinrichtung zugeordnet ist. Je nach Auslegung der Rasteinrichtung kann die von der Rasteinrichtung auf das Dosierorgan ausgeübte Kraft zu klein sein, um das Dosierorgan aus jeder Zwischenstellung zwischen der Nullstellung und der Injektionsstellung bzw. aus jeder Zwischenstellung zwischen zwei Injektionsstellungen selbsttätig und sicher in die Nullstellung oder eine Injektionsstellung zurückzustellen. Um bei einem Injektionsgerät, bei dem das Dosierorgan aufgrund der Auslegung der Rasteinrichtung in eine Zwischenstellung gestellt werden kann, zu vermeiden, dass ein Bediener die nicht vorgesehene Dosis, also eine Dosis, die einer Zwischenstellung entspricht, injizieren kann, wirkt zwischen dem Dosierorgan und dem Gehäuse eine Feder. Sobald der Bediener das Bedienelement in einer Zwischenstellung des Dosierorgans loslässt, wird das Dosierorgan von der Feder aus der Zwischenstellung in eine Injektionsstellung oder die Nullstellung zurückgestellt. Die Injektionsstellung und die Nullstellung sind vorgesehene Stellungen des Dosierorgans. Vorteilhaft wird das Dosierorgan in die nächstniedrige vorgesehene Stellung zurückgestellt, so dass die versehentliche Injektion einer zu hohen Dosis vermieden ist.

Bei üblichen Injektionsgeräten erfolgen das Einstellen der Injektionsdosis und das Injizieren der Injektionsflüssigkeit in unterschiedlichen Bedienbewegungen. Zwischen diesen Bedienbewegungen muss der Bediener das Bedienelement üblicherweise loslassen. Durch die Feder kann auf einfache Weise ein Zurückstellen in die nächstniedrige vorgesehene Stellung des Dosierorgans erreicht werden, wenn der Bediener das Bedienelement nach dem Einstellen der Injektionsdosis und vor dem Auspressen der Injektionsflüssigkeit aus dem Behälter loslässt.

**Das** Dosierorgan **ist** zur Einstellung einer Injektionsdosis um eine Längsmittelachse des Injektionsgeräts drehbar. Dadurch ergeben sich ein kompakter Aufbau und eine einfache Handhabung.

**Die** Rasteinrichtung **wirkt** zwischen einem Zustellteil und dem Gehäuse, wobei das Zustellteil zur Einstellung einer Injektionsdosis um die Längsmittelachse des Injektionsgeräts drehbar ist. **D**as Zustellteil **ist** beim Einstellen einer Injektionsdosis drehfest mit dem Dosierorgan verbunden. Beim Auspressen der Dosis bewegt sich das Zustellteil in Richtung der Längsmittelachse gegenüber dem Gehäuse. Dadurch wird erreicht, dass beim Auspressen der Injektionsflüssigkeit die Rasteinrichtung nicht wirkt und keine Rastschritte für den Bediener hörbar oder spürbar sind. Die Rasteinrichtung kann außerdem so ausgebildet sein, dass ein Zurückdrehen des Zustellteils nach Erreichen einer Injektionsstellung in die nächstniedrige Injektionsstellung nicht mehr möglich ist. Ein einfacher Aufbau ergibt sich **dadurch, dass,** das Zustellteil an mindestens einem Längssteg des Gehäuses geführt ist. **An** dem Längssteg **ist** mindestens ein Rastelement der Rasteinrichtung ausgebildet. Dadurch ergibt sich ein einfacher Aufbau. Der Längssteg bildet gleichzeitig ein Rastelement und die Längsführung für das Zustellteil.

Vorteilhaft wird die Feder beim Einstellen einer Injektionsdosis gespannt. Dabei kann die Feder in Nullstellung des Dosierorgans bereits vorgespannt sein. Es kann jedoch auch vorgesehen sein, dass die Feder in Nullstellung des Dosierorgans weitgehend entspannt ist. Die Feder wirkt insbesondere zwischen dem Dosierorgan und dem Gehäuse. Vorteilhaft ist die Feder direkt mit dem Dosierorgan und dem Gehäuse verbunden. Dadurch ergibt sich eine günstige Einbausituation. Besonders vorteilhaft ist die Feder eine Schraubenfeder, wobei ein erstes Ende der Feder am Dosierorgan und ein zweites Ende der Feder am Gehäuse eingehängt sind. Es kann jedoch auch vorgesehen sein, dass ein Ende der Feder an einem drehfest im Gehäuse gehaltenen Bauteil festgelegt ist. Dadurch kann sich ein vereinfachter Aufbau der Einzelteile des Injektionsgeräts ergeben.

Die Rasteinrichtung gibt vorteilhaft beim Erreichen einer Injektionsstellung eine für den Bediener hörbare und/oder spürbare Rückmeldung. Vorteilhaft übt die Rasteinrichtung in Zwischenstellungen des Dosierorgans keine Kraft auf das Dosierorgan aus. Die Rückstellung des Dosierorgans in eine Injektionsstellung oder die Nullstellung erfolgt dadurch nicht entgegen einer von der Rasteinrichtung ausgeübten Kraft. Dadurch wird eine einfache und sichere Rückstellung erreicht, da die von der Feder ausgeübte Rückstellkraft nicht von einer von der Rasteinrichtung ausgeübten Kraft überlagert wird. Dadurch, dass die Rasteinrichtung in Zwischenstellungen des Dosierorgans keine Kraft auf das Dosierorgan ausübt, können außerdem auch bei geringem zur Verfügung stehendem Bauraum definierte Rastpositionen erreicht werden. Ein einfacher Aufbau ergibt sich, wenn die Rasteinrichtung mindestens eine federnde Raste aufweist, die mit einem Rastelement zusammenwirkt.

Bei einem drehbaren Dosierorgan ist vorgesehen, dass die Injektionsstellungen in Umfangsrichtung um die Längsmittelachse einen Winkelabstand von mindestens etwa 30° zueinander aufweisen. Bei Winkelabständen von etwa 30° oder mehr kann allein aufgrund der geometrischen Gestaltung der Rasteinrichtung eine Rückstellung in vorgesehene Stellungen des Dosierorgans nicht mehr sicher gewährleistet werden. Der Winkelabstand zwischen zwei Injektionsstellungen beträgt vorteilhaft mindestens etwa 45°, insbesondere mindestens etwa 60°. Der Winkelabstand ist vorteilhaft so gewählt, dass ein ganzzahliges Vielfaches des Winkelabstands 360° ergibt.

Das Bedienelement ist vorteilhaft mehrteilig ausgebildet und umfasst einen Betätigungsknopf und die Stellhülse. Die Stellhülse ist fest mit dem Dosierorgan verbunden. Der Betätigungsknopf ist vorteilhaft über einen Mitnehmer mit dem Zustellteil verbunden, wobei der Betätigungsknopf zum Auspressen von Injektionsflüssigkeit aus dem Behälter in Richtung der Längsmittelachse in proximaler Richtung des Injektionsgeräts verschoben wird. Dadurch ergibt sich eine einfache, intuitive Bedienung des Injektionsgeräts. Die "proximale Richtung" bezeichnet dabei die Injektionsrichtung, also in Richtung zu einer Aufnahme für die Injektionsnadel hin bzw. die Richtung, in der die Injektionsflüssigkeit aus dem Behälter ausgepresst wird. Die "distale Richtung" bezeichnet die entgegengesetzte Richtung, also von der Injektionsnadel weg. Das distale Ende des Injektionsgeräts ist das der Injektionsnadel abgewandt liegende Ende. Mit "proximal" wird die Seite des Injektionsgeräts bezeichnet, die bei einer Injektion der Einstichstelle zugewandt liegt, und mit "distal" die Seite, die der Einstichstelle abgewandt liegt. Eine einfache Gestaltung des Injektionsgeräts ergibt sich, wenn der Betätigungsknopf einteilig mit dem Mitnehmer ausgebildet ist. Es kann jedoch auch vorgesehen sein, dass der Mitnehmer mit dem Betätigungsknopf axial fest, aber gegenüber dem Betätigungsknopf drehbar verbunden ist.

Der Betätigungsknopf ist vorteilhaft über eine Kupplung mit der Stellhülse verbunden, die in einer ersten, distalen Position des Betätigungsknopfs eine drehfeste Verbindung zwischen dem Mitnehmer und der Stellhülse herstellt und in einer zweiten, proximalen Position des Betätigungsknopf eines Drehung der Stellhülse gegenüber dem Mitnehmer zulässt. Dadurch kann erreicht werden, dass das Zustellteil beim Einstellen der Injektionsdosis mit dem Dosierorgan mitdreht und beim Auspressen der Injektionsflüssigkeit aus dem Behälter in Längsrichtung des Injektionsgeräts geführt und gegenüber dem Gehäuse nicht drehbar ist, während sich das Dosierorgan um die Längsmittelachse des Injektionsgeräts dreht. Die Drehbewegung des Zustellteils bewirkt vorteilhaft beim Einstellen der Injektionsdosis über eine erste Gewindeverbindung eine axiale Bewegung des Zustellteils um einen ersten Stellweg in Richtung der Längsmittelachse des Injektionsgeräts. Das Zustellteil wird dabei vorteilhaft in distaler Richtung verschoben.

Ein einfacher Aufbau ergibt sich bei einer ersten Ausführungsform des Injektionsgeräts, wenn das Dosierorgan drehbar und in Richtung der Längsmittelachse unverschiebbar im Gehäuse gelagert ist. Dies ist insbesondere für Injektionsgeräte vorteilhaft, bei denen das Dosierorgan zur Einstellung der Maximaldosis um weniger als eine Umdrehung gegenüber dem Gehäuse zu drehen ist. Vorteilhaft besitzt das Injektionsgerät einen Schieber, der ein Gewinde einer zweiten Gewindeverbindung trägt. Die Drehbewegung des Schiebers bewirkt dabei eine Bewegung in distaler Richtung der Längsmittelachse um einen zweiten Stellweg. Der zweite Stellweg, um den sich der Schieber bewegt, ist vorteilhaft mindestens so groß wie der erste Stellweg, um den sich das Zustellteil bewegt.

Um eine genaue Einstellung einer Injektionsdosis zu erlauben, ist bei einer weiteren Ausführungsform des Injektionsgeräts vorteilhaft vorgesehen, dass das Dosierorgan sich beim Einstellen einer Injektionsdosis in Richtung der Längsmittelachse des Injektionsgeräts, vorzugsweise in distaler Richtung, bewegt und dass die Bewegungen von Zustellteil und Dosierorgan in Richtung der Längsmittelachse des Injektionsgeräts sich voneinander unterscheiden. Das Dosierorgan ist vorteilhaft über eine zweite Gewindeverbindung mit dem Gehäuse verbunden, die die Drehbewegung des Dosierorgans in eine Bewegung des Dosierorgans und des Bedienelements in Richtung der Längsmittelachse des Injektionsgeräts um einen zweiten Stellweg bewirkt. Der zweite Stellweg, um den sich das Dosierorgan bewegt, ist dabei insbesondere größer als der erste Stellweg, um den sich das Zustellteil bewegt. Auch das Dosierorgan bewegt sich dabei vorteilhaft in distaler Richtung. Vorteilhaft besitzt das Injektionsgerät einen Schieber, der ein Gewinde einer dritten Gewindeverbindung trägt. Die Drehbewegung des Schiebers bewirkt dabei eine Bewegung in distaler Richtung der Längsmittelachse um einen dritten Stellweg. Der dritte Stellweg, um den sich der Schieber bewegt, ist vorteilhaft mindestens so groß wie der erste Stellweg, um den sich das Zustellteil bewegt. Der Schieber besitzt insbesondere einen Mitnahmeabsatz, der mit einem Mitnahmeabsatz des Zustellteils zusammenwirkt. Der Schieber kann dadurch beim Auspressen von Injektionsflüssigkeit auf das Zustellteil wirken und dieses in proximaler Richtung verschieben.

Ein Ausführungsbeispiel der Erfindung wird im Folgenden anhand der Zeichnung erläutert. Es zeigen:
- Fig. 1: eine Seitenansicht eines Ausführungsbeispiels eines Injektionsgeräts,
- Fig. 2: einen Schnitt entlang der Linie II-II in Fig. 1,
- Fig. 3: einen Abschnitt des Injektionsgerät aus Fig. 1 nach dem Einstellen einer nicht vorgesehenen Menge an Injektionsflüssigkeit,
- Fig. 4: das Injektionsgerät aus Fig. 1 nach dem Einstellen einer vorgesehenen auszupressenden Menge an Injektionsflüssigkeit,
- Fig. 5: einen Schnitt entlang der Linie V-V in Fig. 4,
- Fig. 6: eine vergrößerte Darstellung des distalen Gehäuseteils des Injektionsgeräts aus Fig. 2,
- Fig. 7: eine ausschnittsweise Schnittdarstellung des Injektionsgeräts aus Fig. 5 im Bereich des Bedienelements nach dem Drücken des Betätigungsknopfs,
- Fig. 8: eine Seitenansicht des Injektionsgeräts bei demontiertem Gehäuse,
- Fig. 9: eine Seitenansicht des Mitnehmers des Injektionsgeräts,
- Fig. 10: einen Schnitt entlang der Linie X-X in Fig. 9,
- Fig. 11: einen Schnitt entlang der Linie XI-XI in Fig. 9,
- Fig. 12: eine Seitenansicht des Dosierorgans des Injektionsgeräts,
- Fig. 13: einen Schnitt entlang der Linie XIII-XIII in Fig. 12,
- Fig. 14: eine Seitenansicht des Dosierorgans in Richtung des Pfeils XIV in Fig. 12,
- Fig. 15: eine Seitenansicht eines Innenrohrs des Injektionsgeräts,
- Fig. 16: einen Schnitt entlang der Linie XVI-XVI in Fig. 15,
- Fig. 17: eine vergrößerte Darstellung der Einzelheit XVII in Fig. 16,
- Fig. 18: eine Seitenansicht des Schiebers des Injektionsgeräts,
- Fig. 19: einen Schnitt entlang der Linie XIX-XIX in Fig. 18,
- Fig. 20: einen Schnitt entlang der Linie XX-XX in Fig. 18,
- Fig. 21: eine Seitenansicht des Zustellteils des Injektionsgeräts,
- Fig. 22: einen Schnitt entlang der Linie XXII-XXII in Fig. 21,
- Fig. 23: einen Schnitt entlang der Linie XXIII-XXIII in Fig. 21,
- Fig. 24: eine Seitenansicht des Zustellteils in Richtung des Pfeils XXIV in Fig. 21,
- Fig. 25: eine Seitenansicht der Kolbenstange des Dosierkolbens des Injektionsgeräts,
- Fig. 26: eine Seitenansicht in Richtung des Pfeils XXVI in Fig. 25,
- Fig. 27: einen Schnitt durch das Innenrohr auf der Höhe der Linie XXVII-XXVII in Fig. 15 mit darin angeordnetem Zustellteil in Sperrstellung des Bedienelements,
- Fig. 28: eine Schnittdarstellung entsprechend Fig. 27 mit dem Zustellteil in Injektionsstellung des Bedienelements,
- Fig. 29: eine Seitenansicht eines weiteren Ausführungsbeispiels eines Injektionsgeräts,
- Fig. 30: eine ausschnittsweise Schnittdarstellung des Injektionsgeräts aus Fig. 29 entlang der Linie XXX-XXX in Fig. 29,
- Fig. 31: eine Seitenansicht des Injektionsgeräts aus Fig. 1 nach dem Einstellen einer vorgesehenen auszupressenden Menge an Injektionsflüssigkeit,
- Fig. 32: eine ausschnittsweise Schnittdarstellung des Injektionsgeräts aus Fig. 31 entlang der Linie XXXII-XXXII in Fig. 31,
- Fig. 33: eine ausschnittsweise Schnittdarstellung des Injektionsgeräts aus Fig. 31 entlang der Linie XXXII-XXXII in Fig. 31 nach dem Drücken des Betätigungsknopfs,
- Fig. 34: eine Seitenansicht des Injektionsgeräts bei demontiertem Gehäuse,
- Fig. 35: eine Seitenansicht in Richtung des Pfeils XXXV in Fig. 34,
- Fig. 36: eine Seitenansicht in Richtung des Pfeils XXXVI in Fig. 35,
- Fig. 37: eine perspektivische Darstellung des Dosierorgans des Injektionsgeräts aus Fig. 29,
- Fig. 38: eine Seitenansicht des Dosierorgans aus Fig. 37,
- Fig. 39: einen Schnitt entlang der Linie XXXIX-XXXIX in Fig. 38,
- Fig. 40: eine Seitenansicht in Richtung des Pfeils XL in Fig. 38,
- Fig. 41: eine Seitenansicht auf die Skala des Dosierorgans aus Fig. 37,
- Fig. 42: eine Seitenansicht in Richtung des Pfeils XLII in Fig. 41,
- Fig. 43: eine Seitenansicht auf die Skala eines Ausführungsbeispiels des Dosierorgans,
- Fig. 44: eine Seitenansicht in Richtung des Pfeils XLIV in Fig. 43,
- Fig. 45: eine Seitenansicht in Richtung des Pfeils XLV in Fig. 44,
- Fig. 46: eine perspektivische Darstellung des Betätigungsknopfs des Injektionsgeräts aus Fig. 29,
- Fig. 47: eine Seitenansicht des Betätigungsknopfs aus Fig. 46,
- Fig. 48: einen Schnitt entlang der Linie XLVIII-XLVIII in Fig. 47,
- Fig. 49: einen Schnitt entlang der Linie XLIX-XLIX in Fig. 47,
- Fig. 50: eine perspektivische Darstellung des Kolbenstangenrings des Injektionsgeräts aus Fig. 29,
- Fig. 51: eine Seitenansicht des Kolbenstangenrings aus Fig. 50,
- Fig. 52: eine Seitenansicht in Richtung des Pfeils LII-LII in Fig. 51,
- Fig. 53: eine Ansicht von oben in Richtung des Pfeils LIII-LIII in Fig. 51,
- Fig. 54: eine perspektivische Darstellung eines Ausführungsbeispiels eines Kolbenstangenrings des Injektionsgeräts aus Fig. 29,
- Fig. 55: eine Ansicht des Kolbenstangenrings aus Fig. 54 von oben,
- Fig. 56: eine perspektivische Darstellung des Schiebers des Injektionsgeräts aus Fig. 29,
- Fig. 57: eine Seitenansicht des Schiebers aus Fig. 56,
- Fig. 58: eine Seitenansicht in Richtung des Pfeils LVIII in Fig. 57,
- Fig. 59 und 60: perspektivische Darstellungen des Zustellteils des Injektionsgeräts aus Fig. 29,
- Fig. 61: eine Seitenansicht des Zustellteils aus den Fig. 59 und 60,
- Fig. 62: einen Schnitt entlang der Linie LXII-LXII in Fig. 61,
- Fig. 63: eine Seitenansicht in Richtung des Pfeils LXIII in Fig. 61,
- Fig. 64: eine Seitenansicht des oberen Gehäuseteils des Injektionsgeräts aus Fig. 29,
- Fig. 65: einen Schnitt entlang der Linie LXV-LXV in Fig. 64,
- Fig. 66: einen Schnitt entlang der Linie LXVI-LXVI in Fig. 65,
- Fig. 67: eine Seitenansicht des Injektionsgeräts aus Fig. 29 nach dem Einstellen einer nicht vorgesehenen Menge an Injektionsflüssigkeit,
- Fig. 68: einen Schnitt entlang der Linie LXVIII-LXVIII in Fig. 67,
- Fig. 69: eine Seitenansicht des Injektionsgeräts aus Fig. 29 nach dem Einstellen einer vorgesehenen auszupressenden Menge an Injektionsflüssigkeit,
- Fig. 70: einen Schnitt entlang der Linie LXX-LXX in Fig. 69.

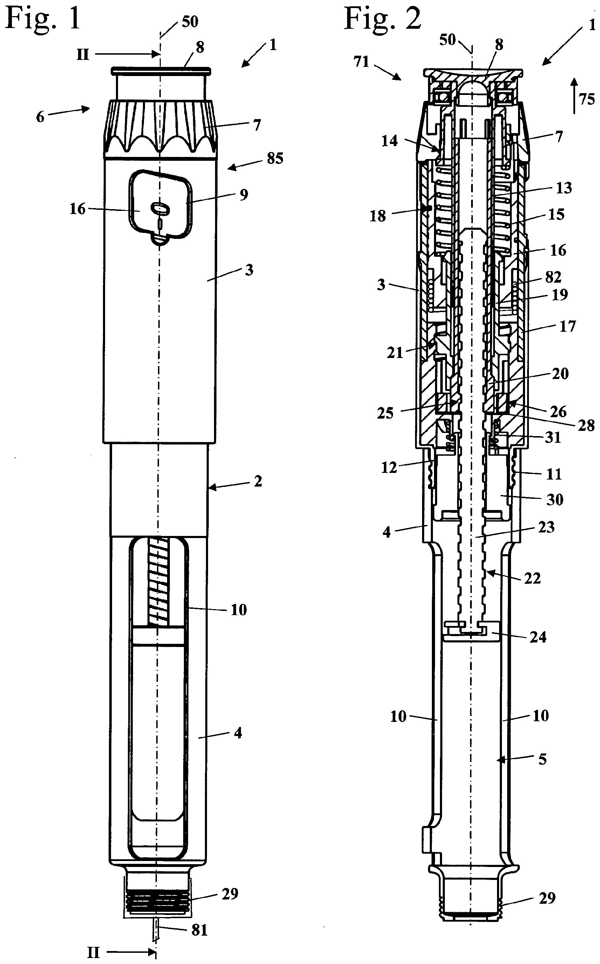

Das in Fig. 1 gezeigte Injektionsgerät 1 besitzt ein Gehäuse 2, das ein oberes, distales Gehäuseteil 3 und einen an der proximalen Seite des oberen Gehäuseteils 3 angeordneten Halter 4 umfasst. An seinem proximalen Ende besitzt der Halter 4 ein Außengewinde 29, an dem eine in Fig. 1 schematisch gezeigte Injektionsnadel 81 aufgeschraubt werden kann. Im Halter 4 ist eine in Fig. 2 gezeigte Aufnahme 5 für einen Behälter mit Injektionsflüssigkeit ausgebildet. Der Behälter mit Injektionsflüssigkeit ist in den Figuren zum ersten Ausführungsbeispiel nicht gezeigt. Wie Fig. 1 zeigt, weist der Halter 4 mindestens eine Aussparung 10 auf, durch die der Behälter mit Injektionsflüssigkeit sichtbar ist. Dadurch kann der Bediener leicht erkennen, ob noch Injektionsflüssigkeit im Behälter vorhanden ist. Wie Fig. 2 zeigt, sind zwei einander gegenüberliegend angeordnete Aussparungen 10 am Halter 4 vorgesehen.

Wie Fig. 1 zeigt, ist am distalen Ende des Gehäuseteils 3 ein Bedienelement 6 angeordnet, das eine Stellhülse 7 und einen an der distalen Seite der Stellhülse 7 angeordneten Betätigungsknopf 8 besitzt. Benachbart zur Stellhülse 7 weist das Gehäuseteil 3 ein Sichtfenster 9 auf, durch das eine auf einem Dosierorgan 16 aufgebrachte Skala erkennbar ist. Das Dosierorgan 16 ist im Gehäuseteil 3 angeordnet. In Fig. 1 zeigt die Skala eine "0", die dem Bediener signalisiert, dass keine Mengeneinstellung vorgenommen wurde. Das Dosierorgan 16 befindet sich in einer Nullstellung 85, in der keine Dosis eingestellt ist.

Fig. 2 zeigt den Aufbau des Injektionsgeräts 1 im Einzelnen. Das Injektionsgerät 1 besitzt einen Mitnehmer 13, der im Wesentlichen hülsenförmig ausgebildet ist und der axial fest mit dem Betätigungsknopf 8 des Bedienelements 6 verbunden ist. Der Begriff "axial" bezieht sich dabei jeweils auf die Richtung einer Längsmittelachse 50 des Injektionsgeräts 1. Der Betätigungsknopf 8 ist mit dem Mitnehmer 13 über eine Schnappverbindung verbunden, die eine Drehung des Betätigungsknopfs 8 gegenüber dem Mitnehmer 13 zulässt. Der Mitnehmer 13 ist über eine Kupplung 14 mit der Stellhülse 7 des Bedienelements 6 verbunden. Bei der in Fig. 2 gezeigten ersten, distalen Position 71 des Betätigungsknopfs 8 ist die Kupplung 14 geschlossen. Die Stellhülse 7 des Bedienelements 6 ist drehfest mit dem Mitnehmer 13 verbunden. Die Stellhülse 7 ist fest mit dem Dosierorgan 16 verbunden, das auch als Einstellglied oder Skalenrohr bezeichnet wird. Der Mitnehmer 13 ist drehfest mit einem Zustellteil 20 verbunden, das über eine erste Gewindeverbindung 25 mit einer Kolbenstange 23 eines Dosierkolbens 22 verbunden ist. Die Kolbenstange 23 trägt an ihrem proximalen Ende eine Kolbenscheibe 24, die zur Anlage an einem Stopfen des Behälters mit Injektionsflüssigkeit dient und über die die Injektionsflüssigkeit aus dem Behälter ausgepresst wird.

Die Kolbenstange 23 ist drehfest in einem Kolbenstangenring 30 gehalten. Der Kolbenstangenring 30 ist axial verschiebbar im Injektionsgerät 1 angeordnet. In der in Fig. 2 gezeigten Stellung, wenn kein Behälter in die Aufnahme 5 eingesetzt ist, wird der Kolbenstangenring 30 von einer Druckfeder 31 in seine proximale Position gedrückt. In dieser Position ist der Kolbenstangenring 30 gegenüber dem Gehäuseteil 3 drehbar. Wird ein Behälter in die Aufnahme 5 eingesetzt und der Halter 4 an einem Befestigungsgewinde 11 mit dem Gehäuseteil 3 verbunden, so drückt der Behälter den Kolbenstangenring 30 in distale Richtung. Das Injektionsgerät 1 besitzt ein Innenrohr 17, das ein Teil des Gehäuses 2 ist und das drehfest und in axialer Richtung fest mit dem Gehäuseteil 3 verbunden ist. An seinem distalen Ende weist der Kolbenstangenring 30 eine Kontur 12 auf, die auf eine Kontur des Innenrohrs 17 abgestimmt ist. In seiner distalen Stellung ist der Kolbenstangenring 30 über die genannten Konturen drehfest mit dem Innenrohr 17 und damit auch drehfest mit dem Gehäuseteil 3 verbunden. Bei in die Aufnahme 5 eingesetztem Behälter ist dadurch die Kolbenstange 23 drehfest im Gehäuseteil 3 gehalten. Durch die drehfeste Verbindung von Kolbenstange 23 und Gehäuseteil 3 bewirkt eine Drehung des Zustellteils 20 eine Bewegung des Zustellteils 20 in distaler Richtung, also in Richtung des Pfeils 75 in Fig. 2. Zwischen dem Zustellteil 20 und dem Innenrohr 17 ist eine Rasteinrichtung 26 gebildet, die Raststellungen des Zustellteils 20 definiert. Bei der in Fig. 2 gezeigten Stellung des Zustellteils 20 liegt das Zustellteil 20 an einem am Innenrohr 17 gebildeten Anschlag 28 an, der die Position des Zustellteils 20 in axialer Richtung definiert.

Das Dosierorgan 16 ist über eine zweite Gewindeverbindung 18 mit dem Innenrohr 17 verbunden. Das Innenrohr 17 ist fest mit dem Gehäuseteil 3 verbunden. Das Innenrohr 17 könnte auch einteilig mit dem Gehäuseteil 3 ausgebildet sein, allerdings wird dadurch die Herstellung des Injektionsgeräts 1 sehr aufwendig. Das Dosierorgan 16 ist drehfest und axial verschiebbar mit einem Schieber 19 verbunden, der ins Innere des Dosierorgans 16 ragt. Der Schieber 19 ist über eine dritte Gewindeverbindung 21 mit dem Innenrohr 17 verbunden. Zwischen dem Mitnehmer 13 und dem Dosierorgan 16 wirkt eine Druckfeder 15, die den Betätigungsknopf 8 in seine erste Position 71 drückt. Zwischen dem Dosierorgan 16 und dem Gehäuse 3 wirkt eine Feder 82. Die Feder 82 ist vorteilhaft als Torsionsfeder ausgebildet. Im Ausführungsbeispiel ist die Feder 82 eine Schraubenzugfeder, die beim Einstellen einer Injektionsdosis sowohl gelängt als auch um die Längsmittelachse 50 des Injektionsgeräts 1 verdreht wird.

Zum Einstellen der auszupressenden Menge an Injektionsflüssigkeit dreht der Bediener das Bedienelement 6, bis die gewünschte Dosis im Sichtfenster 9 erscheint. Dabei dreht sich die Stellhülse 7. Das drehfest mit der Stellhülse 7 verbundene Dosierorgan 16 dreht sich dadurch gegenüber dem oberen Gehäuseteil 3 und dem Innenrohr 17. Aufgrund seiner Drehbewegung wird das Dosierorgan 16 über die zweite Gewindeverbindung 18 in distaler Richtung, also in Richtung des Pfeils 75 verschoben. Das Bedienelement 6 und der axial fest mit dem Betätigungsknopf 8 des Bedienelements 6 verbundene Mitnehmer 13 bewegen sich mit dem Dosierorgan 16. Das Bedienelement 6, der Mitnehmer 13 und das Dosierorgan 16 bewegen sich gemeinsam in distaler Richtung und drehen sich dabei aufgrund der zweiten Gewindeverbindung 18 um die Längsmittelachse 50.

Über die drehfeste Verbindung zwischen Mitnehmer 13 und Zustellteil 20 wird auch das Zustellteil 20 gegenüber dem oberen Gehäuseteil 3 gedreht. Über die erste Gewindeverbindung 25 bewegt sich auch das Zustellteil 20 in distaler Richtung. Der Schieber 19 bewegt sich ebenfalls in distaler Richtung, da der Schieber 19 drehfest mit dem Dosierorgan 16 verbunden ist. Auch der Schieber 19 und das Zustellteil 20 bewegen sich mit einer kombinierten Dreh- und Längsbewegung, wobei der Weg, den Schieber 19 und Zustellteil 20 in Richtung der Längsmittelachse 50 zurücklegen, über die erste Gewindeverbindung 25 bzw. über die dritte Gewindeverbindung 21 festgelegt sind. Es kann auch vorgesehen sein, dass der Schieber 19 über eine dritte Gewindeverbindung mit dem Dosierorgan 16 verbunden ist und drehfest mit dem Gehäuseteil 3 verbunden ist.

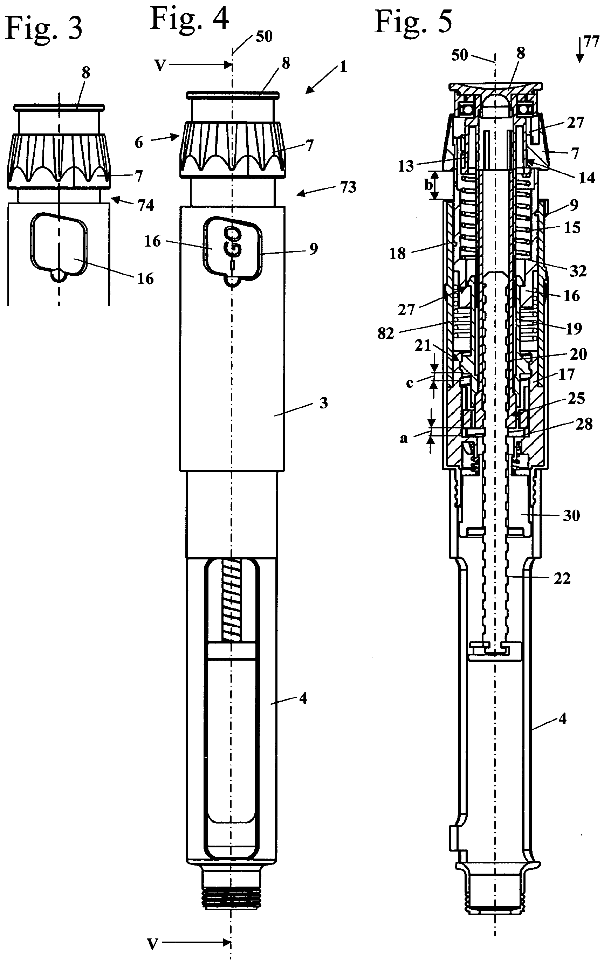

Die Fig. 3 zeigt das Injektionsgerät 1 nach dem Einstellen einer vom Hersteller nicht vorgesehenen Dosis an Injektionsflüssigkeit. Das Dosierorgan 16 befindet sich in einer Zwischenstellung 74, die im Folgenden noch näher erläutert wird.

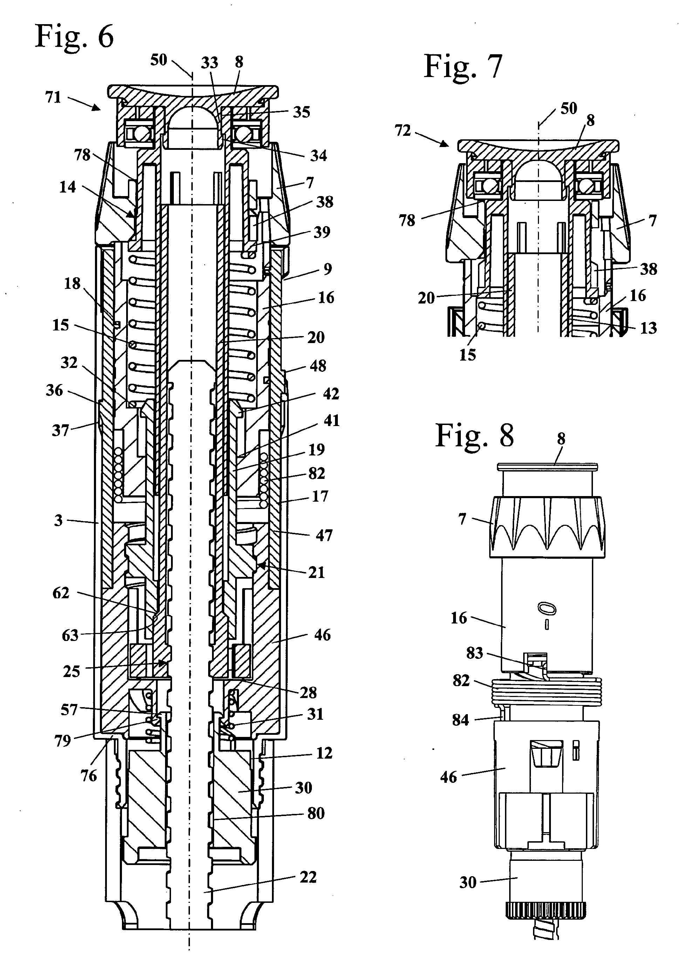

Die Figuren 4 und 5 zeigen das Injektionsgerät 1 nach dem Einstellen einer vorgesehenen auszupressenden Menge an Injektionsflüssigkeit. Das Zustellteil 20 hat sich um einen ersten Stellweg a in distaler Richtung bewegt. Nach dem Einstellen der auszupressenden Menge an Injektionsflüssigkeit hat sich die Stirnseite des Zustellteils 20 um den ersten Stellweg a vom Anschlag 28 entfernt. Das Bedienelement 6 mit der Stellhülse 7 und dem Betätigungsknopf 8 hat sich um einen zweiten Stellweg b in distaler Richtung bewegt. Der zweite Stellweg b ist im Ausführungsbeispiel zwischen der proximalen Stirnseite der Stellhülse 7 und der distalen Stirnseite des Gehäuseteils 3 gemessen. Der zweite Stellweg b ist deutlich größer als der erste Stellweg a. Im Ausführungsbeispiel beträgt der zweite Stellweg b ein Vielfaches, beispielsweise etwa das 3fache des Stellwegs a. Die unterschiedlichen Stellwege a und b ergeben sich durch unterschiedliche Steigungen der ersten Gewindeverbindung 25 und der zweiten Gewindeverbindung 18. Auch das Dosierorgan 16 hat sich um den zweiten Stellweg b in distaler Richtung bewegt. Der Schieber 19 hat sich um einen dritten Stellweg c in distaler Richtung bewegt. Der Stellweg c kann gleich groß sein wie der Stellweg a. Es kann jedoch auch vorgesehen sein, dass der Stellweg c größer als der Stellweg a ist. Der dritte Stellweg c ist in Fig. 5 an der proximalen Stirnseite des Abschnitts des Schiebers 19, der das Gewinde trägt, eingezeichnet, und zwar gegenüber der Position dieser Stirnseite in Fig. 2. Die Feder 82 wurde beim Einstellen der auszupressenden Menge an Injektionsflüssigkeit gespannt. Dabei wurde die Feder 82 um den zweiten Stellweg b gelängt. Gleichzeitig wurden die in Fig. 8 gezeigten Enden 83 und 84 der Feder 82 aufgrund der Drehung des Dosierorgans 16 gegenüber dem Gehäuse 2 um die Längsmittelachse 50 gegeneinander verdreht.

Die maximal einzustellende Menge an Injektionsflüssigkeit ist durch den Weg vorgegeben, den der Betätigungsknopf 6 und das Dosierorgan 16 sich in distaler Richtung bewegen können. Dieser Weg wird durch einen zwischen dem Dosierorgan 16 und dem Schieber 19 gebildeten Anschlag 27 (Fig. 5) begrenzt. Wie Fig. 4 zeigt, besitzt das Dosierorgan 16 an seinem proximalen Ende einen nach innen gerichteten Absatz 41. Dieser Absatz 41 wird von einem Rastrand 42 am Schieber 19 in axialer Richtung hintergriffen. Der Absatz 41 bildet mit dem Rastrand 42 den Anschlag 27. Sobald der Rastrand 42 am Absatz 41 anliegt, ist die maximal einstellbare Menge an Injektionsflüssigkeit erreicht. Der Abstand zwischen Absatz 41 und Rastrand 42 bei dem in den Figuren 1 und 2 gezeigten Zustand des Injektionsgeräts 1 entspricht dem zweiten Stellweg b abzüglich dem ersten Stellweg a.

Wie Fig. 6 zeigt, besitzt das erste Gehäuseteil 3 eine Rastvertiefung 37, die im Ausführungsbeispiel umlaufend ausgebildet ist. In die Rastvertiefung 37 ragt eine am Innenrohr 17 ausgebildete, radial nach außen ragende Raste 36, die das Innenrohr 17 in Richtung der Längsmittelachse 50 des Injektionsgeräts 1 im Gehäuseteil 3 sichert. An seinem proximalen Ende liegt das Innenrohr 17 an einer Schulter 76 des Gehäuseteils 3 an. Zur Drehlagensicherung besitzt das Innenrohr 17 einen nach außen ragenden Zapfen 48, der benachbart zum Sichtfenster 9 am Gehäuseteil 3 einrastet.

Fig. 6 zeigt auch die Abstützung der Druckfeder 15. Die Druckfeder 15 stützt sich mit ihrem proximalen Ende an einer Schulter 32 des Dosierorgans 16 und mit ihrem distalen Ende an einem am Mitnehmer 13 ausgebildeten Rand 39 ab. Der Rand 39 ragt von einem hülsenförmigen Abschnitt des Mitnehmers 13 nach außen. Benachbart zum Rand 39 ist an der distalen Seite des Rands 39 eine Außenverzahnung 38 am Mitnehmer 13 angeordnet. Die Außenverzahnung 38 wirkt mit einer nicht gezeigten Innenverzahnung an der Stellhülse 7 zusammen und bildet mit dieser die Kupplung 14. Bei der in Fig. 6 gezeigten, nicht betätigten Stellung des Betätigungsknopfs 8 ist die Kupplung 14 geschlossen und stellt eine drehfeste Verbindung zwischen Mitnehmer 13 und Stellhülse 7 her. Die Druckfeder 15 drückt den Mitnehmer 13 in Richtung auf die geschlossene Stellung der Kupplung 14. Dadurch wird der Betätigungsknopf 8 in Richtung auf seine distale Position 71 gedrückt.

Nach dem Einstellen der zu injizierenden Dosis kann eine Injektion ausgelöst werden. Hierzu wird der Betätigungsknopf 8 in Richtung des Pfeils 77 in Fig. 5, also in proximaler Richtung, gedrückt. Dadurch bewegt sich der Betätigungsknopf 8 entgegen der Kraft der Druckfeder 15 in Richtung der Längsmittelachse 50 in die Stellhülse 7 hinein, bis der Betätigungsknopf 8 an einem Anschlag 78 der Stellhülse 7 anliegt. Fig. 7 zeigt den Betätigungsknopf 8 in seiner zweiten, proximalen Position 72. In dieser Position hat sich die Außenverzahnung 38 des Mitnehmers 13 aus dem Bereich der Stellhülse 7 bewegt. Dadurch ist die Stellhülse 7 gegenüber Mitnehmer 13 und dem Betätigungsknopf 8 drehbar. Die Kupplung 14 ist offen. Bei weiterem Drücken des Betätigungsknopfs 8 in Richtung des Pfeils 77 in Fig. 5 wird das Dosierorgan 16 in das Innenrohr 17 gedrückt und verschiebt sich dabei in proximaler Richtung. Dabei dreht sich das Dosierorgan 16 aufgrund der zweiten Gewindeverbindung 18. Aufgrund der Drehung des Dosierorgans 16 wird auch der Schieber 19 gedreht und bewegt sich dadurch in proximaler Richtung. Die Drehung des Dosierorgans 16 gegenüber dem Gehäuse 3 wird von der Feder 82 unterstützt. Der Schieber 19 besitzt einen Mitnahmeabsatz 62, der an einem Mitnahmeabsatz 63 des Zustellteils 20 anliegt. Über die Mitnahmeabsätze 62 und 63 drückt der Schieber 19 bei seiner Bewegung in proximaler Richtung auf das Zustellteil 20 und bewegt dieses ebenfalls in proximaler Richtung. Das Zustellteil 20 ist drehfest mit dem Mitnehmer 13 verbunden, der mit dem nicht rotierenden Betätigungsknopf 8 axial fest verbunden ist. Eine Drehung des Zustellteils 20 wird von der Rasteinrichtung 26 verhindert, die beim Einstellen der Dosis in eine Raststellung gestellt worden ist. Der rotierende Schieber 19 kann das Zustellteil 20 dadurch nicht mitdrehen. Da das Zustellteil 20 nicht rotiert und auch der Dosierkolben 22 über den Kolbenstangenring 30 drehfest mit dem Gehäuseteil 3 verbunden ist, sind das Zustellteil 20 und der Dosierkolben 22 fest miteinander verbunden und bewegen sich gemeinsam in proximaler Richtung, bis das Zustellteil 20 am Anschlag 28 anliegt und die eingestellte Menge an Injektionsflüssigkeit vollständig aus dem Behälter ausgepresst wurde.

Das Injektionsgerät 1 ist zur Injektion vorgegebener Dosen von Injektionsflüssigkeit vorgesehen. Das Dosierorgan 16 besitzt mindestens eine in Fig. 4 gezeigte Injektionsstellung 73, in der eine konstruktiv vorgegebene, vorgesehene Menge an Injektionsflüssigkeit eingestellt ist. In Injektionsstellungen 73 rastet die Rasteinrichtung 26 ein. Das Dosierorgan 16 kann auch in mindestens eine Zwischenstellung 74 gestellt werden, die in Fig. 3 gezeigt ist. In einer Zwischenstellung 74 des Dosierorgans 16 ist eine nicht vorgesehene Menge an Injektionsflüssigkeit eingestellt. In Zwischenstellungen 74 des Dosierorgans 16 rastet die Rasteinrichtung 26 nicht ein. Sind nicht vorgesehene Dosen von Injektionsflüssigkeit eingestellt, so wird das Dosierorgan 16 von der Feder 82 in die nächstniedrige Injektionsstellung 73 oder die Nullstellung 85 zurückgestellt, sobald der Bediener die Stellhülse 7 loslässt.

Wie Fig. 6 zeigt, ist das Innenrohr 17 des Gehäuses 2 aus einem proximalen Teil 46 und einem distalen Teil 47 aufgebaut, die fest miteinander verbunden sind. Das Innenrohr 17 kann auch einteilig hergestellt werden. Allerdings ergibt sich dadurch eine deutlich aufwendigere Herstellung des Innenrohrs 17. Um die Herstellung weiter zu vereinfachen, kann es vorteilhaft sein, das Innenrohr 17 aus mehr als zwei Einzelteilen auszubilden. Wie Fig. 8 zeigt, wirkt die Feder 82 zwischen dem distalen Teil 46 des Innenrohrs 17 und dem Dosierorgan 16. Dabei ist ein erstes Ende 83 der Feder 82 am Dosierorgan 16 festgelegt und ein zweites Ende 84 am proximalen Teil 46 des Innenrohrs 17. Die Enden 83 und 84 sind dabei vorteilhaft in entsprechenden Aussparungen von Dosierorgan 16 und proximalem Teil 46 eingehängt.

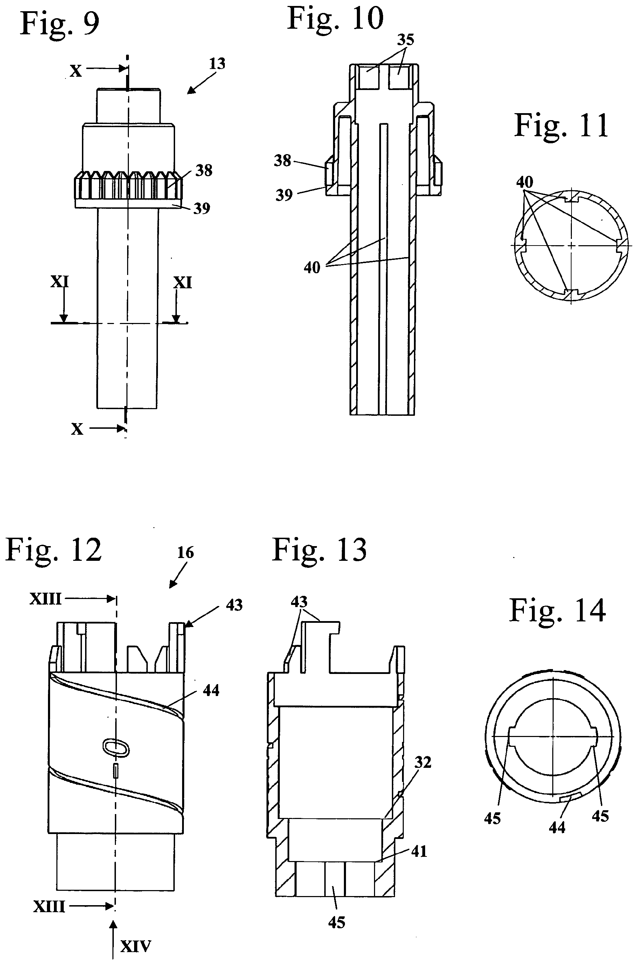

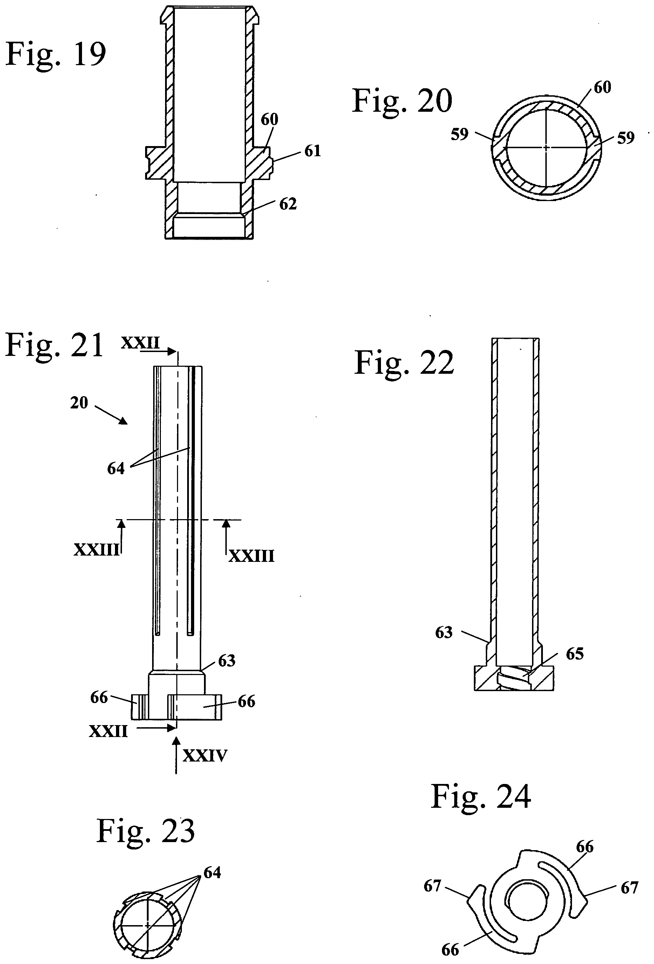

Die Figuren 9 bis 27 zeigen die Bauteile des Injektionsgeräts 1 im Einzelnen. In den Figuren 9 bis 11 ist der Mitnehmer 13 gezeigt. Zur Verbindung mit dem Betätigungsknopf 8 besitzt der Mitnehmer 13 an seinem distalen Ende innenliegende Rasterhöhungen 35, die einen an einem Stutzen 33 des Betätigungsknopfs 8 gebildeten, in Fig. 6 gezeigten Rastrand 34 hintergreifen und dadurch den Betätigungsknopf 8 in axialer Richtung mit dem Mitnehmer 13 verbinden. Der hülsenförmige Mitnehmer 13 besitzt an seinem Innenumfang im Ausführungsbeispiel vier in axialer Richtung verlaufende Führungsstege 40. Die Führungsstege 40 sind auf in Fig. 22 gezeigte Längsnuten 64 des Zustellteils 20 angepasst und greifen in diese ein. Die Führungsstege 40 stellen mit den Längsnuten 64 die drehfeste Verbindung zwischen Mitnehmer 13 und Zustellteil 20 her. Die Führungsstege 40 sind in Richtung der Längsmittelachse 50 des Injektionsgeräts 1 in den Längsnuten 64 frei verschiebbar.

Die Figuren 12 bis 14 zeigen das Dosierorgan 16, das auch als Skalenrohr oder Einstellglied bezeichnet wird. Das Dosierorgan 16 ist hülsenförmig ausgebildet und besitzt an seinem Außenumfang ein Außengewinde 44. Das Außengewinde 44 ist als wendelförmig am Außenumfang des Dosierorgans 16 verlaufende Nut ausgebildet. An seinem distalen Ende trägt das Dosierorgan 16 eine Verbindungskontur 43, die aus haken- und rampenförmigen Elementen gebildet ist, die eine drehfeste Verbindung mit der Stellhülse 7 herstellen. Wie die Figuren 13 und 14 zeigen, besitzt das Dosierorgan 16 an seinem proximalen Ende zwei Führungsnuten 45, die parallel zur Längsmittelachse 50 verlaufen. Die Führungsnuten 45 sind einander gegenüberliegend angeordnet und wirken mit Längsstegen 59 des Schiebers 19 zusammen, die in den Figuren 19 und 21 gezeigt sind. Über die Längsstege 59, die in den Führungsnuten 45 geführt sind, ergibt sich eine drehfeste Verbindung zwischen Dosierorgan 16 und Schieber 19. Die Längsstege 59 sind in Richtung der Längsmittelachse 50 frei in den Führungsnuten 45 beweglich, so dass der Schieber 19 gegenüber dem Dosierorgan 16 in Richtung der Längsmittelachse 50 verschiebbar ist.

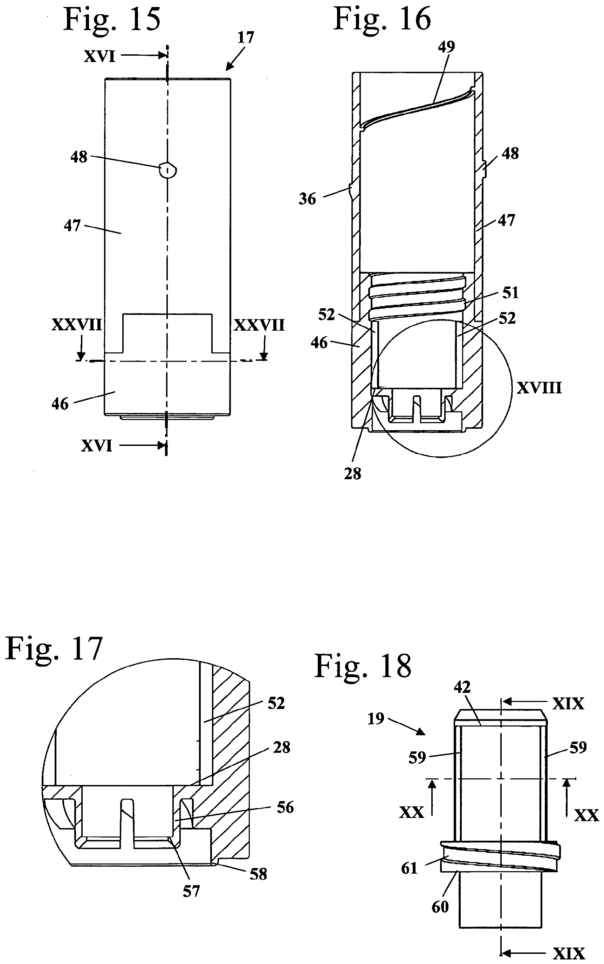

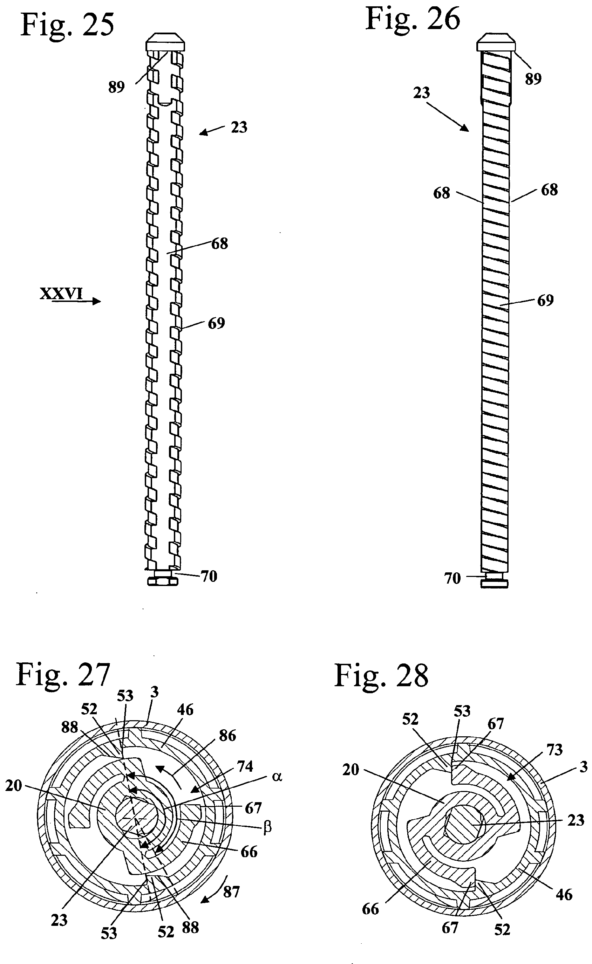

In den Figuren 15 bis 17 ist das Innenrohr 17 gezeigt. Das Innenrohr 17 ist zweiteilig ausgeführt und besteht aus dem proximalen Teil 46 und dem distalen Teil 47, die fest miteinander verbunden sind. Im distalen Teil 47 des Innenrohrs 17 ist ein Innengewinde 49 angeordnet, das aus einem spiralförmig am Innenumfang verlaufenden Steg gebildet ist. Das Innengewinde 49 ist durch einen einzigen Gewindegang gebildet. Es kann vorgesehen sein, das Innengewinde 49 nur durch einen oder mehrere Teilabschnitte eines Gewindegangs auszubilden. Das Innengewinde 49 wirkt mit dem Außengewinde 44 des Dosierorgans 16 zusammen und bewirkt eine axiale Verschiebung des Dosierorgans 16 bei einer Drehung des Dosierorgans 16. Im proximalen Teil 46 des Innenrohrs 17 ist ein Innengewinde 51 ausgebildet, das mit einem in Fig. 18 gezeigten Außengewinde 61 des Schiebers 19 zusammenwirkt. Das Innengewinde 51 bildet mit dem Außengewinde 61 die dritte Gewindeverbindung 21. An der proximalen Seite des Innengewindes 51 schließen sich Längsstege 52 an. Wie Fig. 27 zeigt, sind im Ausführungsbeispiel insgesamt zwei Längsstege 52 vorgesehen, die etwa einander gegenüberliegend angeordnet sind. Die Längsstege 52 definieren mit den beiden einander gegenüberliegenden Rasten 67 des Zustellteils 20 die Raststellungen der Rasteinrichtung und damit die Injektionsstellungen 73. Die beiden Injektionsstellungen 73 besitzen im Ausführungsbeispiel einen Winkelabstand α von 180°. Der Winkelabstand α beträgt vorteilhaft mindestens etwa 30°, insbesondere mindestens etwa 45°, besonders vorteilhaft mindestens 60°. Die Längsstege 52 besitzen in Umfangsrichtung einen in Fig. 27 gezeigten Winkelabstand β zueinander, der im Ausführungsbeispiel etwas weniger als 180°, beispielsweise etwa 160° bis 175°, beträgt. Der Winkelabstand β entspricht dem Winkelbereich, in dem das Dosierorgan 16 zwischen zwei Injektionsstellungen 73 oder einer Injektionsstellung 73 und einer Nullstellung 85 in Zwischenstellungen 74 gestellt werden kann. Vorteilhaft übt in diesem Winkelbereich die Rasteinrichtung 26 keine Kraft auf das Zustellteil 20 und damit auf das Dosierorgan 16 aus. Es kann auch eine andere Anzahl von Längsstegen 52 und/oder Rasten 67 vorteilhaft sein. Beispielsweise können vier Längsstege 52 und zwei Rasten 67 vorgesehen sein, die so angeordnet sind, dass sich ein Winkelabstand α von 90° zwischen den Injektionsstellungen 73 ergibt.

Im Ausführungsbeispiel sind die Längsstege 52 und die Rasten 67 so gestaltet, dass ein Zurückdrehen des Bedienelements 6 aus einer Raststellung in eine Stellung, die einer geringeren Menge an Injektionsflüssigkeit zugeordnet ist, nicht möglich ist. Es kann jedoch auch vorgesehen sein, dass die Form der Längsstege 52 und Rasten 67 ein Zurückdrehen des Bedienelements 6 erlauben, beispielsweise durch in Umfangsrichtung symmetrische Gestaltung.

Wie Fig. 17 zeigt, ragt am proximalen Ende des Innenrohrs 17 ein Zentrierrand 58 in proximale Richtung. Der Zentrierrand 58 ragt in eine proximale Öffnung des Gehäuseteils 3 und stellt einen festen Sitz des Innenrohrs 17 im Gehäuseteil 3 sicher. An der proximalen Seite des Innenrohrs 17 ragen außerdem Haltestutzen 56 in proximale Richtung, an deren proximalen Ende radial nach innen ragende Rastränder 57 angeformt sind. Die Rastränder 57 wirken mit einem Rastrand 79 des Kolbenstangenrings 30 zusammen, der in Fig. 5 gezeigt ist. Der Rastrand 79 stellt mit dem Rastrand 57 eine axiale Sicherung für den Kolbenstangenring 30 dar. Wie Fig. 5 zeigt, drückt die zweite Druckfeder 31 den Kolbenstangenring 30 in seine proximale Position, bis der Rastrand 79 am Rastrand 57 anliegt. In dieser Position kann der Bediener das Gehäuseteil 3 gegenüber dem Kolbenstangenring 30 drehen, um den Dosierkolben 22 in distale Richtung zu bewegen. Dies ist beim Auswechseln eines Behälters für Injektionsflüssigkeit vorgesehen.

In den Figuren 18 bis 20 ist der Schieber 19 im Einzelnen gezeigt. An seinem distalen Ende besitzt der Schieber 19 den Rastrand 42. Wie die Figuren 18 und 19 zeigen, ist das Außengewinde 61 an einem Ringsteg 60 ausgebildet, der radial nach außen ragt. Auch der Schieber 19 ist im Wesentlichen hülsenförmig ausgebildet.

Die Figuren 21 bis 24 zeigen das Zustellteil 20. An seinem proximalen Ende besitzt das Zustellteil 20 zwei Rastarme 66, die in Fig. 24 gezeigt sind. An ihrem freien Ende besitzen die Rastarme 66 jeweils eine Raste 67, die radial nach außen weist. Die Rastarme 66 verlaufen etwa in Umfangsrichtung und sind radial nach außen federnd ausgebildet. Fig. 22 zeigt ein am proximalen Ende des Zustellteils 20 ausgebildetes Innengewinde 65, das mit dem Dosierkolben 22 zusammenwirkt. Das Innengewinde 65 und die Rastarme 65 sind im gleichen Längsabschnitt des Zustellteils 20 angeordnet.

Wie die Figuren 25 und 26 zeigen, besitzt die Kolbenstange 23 ein Außengewinde 69, das mit dem Innengewinde 65 des Zustellteils 20 zusammenwirkt und mit diesem die erste Gewindeverbindung 25 bildet. An ihren gegenüberliegenden Längsseiten besitzt die Kolbenstange 23 Abflachungen 68, die zur Sicherung der Drehlage der Kolbenstange 23 mit entsprechenden Abflachungen einer in Fig. 5 gezeigten Öffnung 80 im Kolbenstangenring 30 zusammenwirken. An ihrem proximalen Ende besitzt die Kolbenstange 23 eine Befestigungsnut 70, an der die Kolbenscheibe 24 gehalten ist. An ihrem distalen Ende besitzt die Kolbenstange 23 einen Anschlag 89. Am distalen Ende der Kolbenstange 23 endet das Außengewinde 69 in einer Kontur, die im Ausführungsbeispiel einen runden Querschnitt mit einem Durchmesser aufweist, der größer als der Außendurchmesser des Außengewindes 69 ist. Die proximale Fläche dieser Kontur bildet den Anschlag 89 zum Innengewinde 68 des Zustellteils 20. Am Anschlag 89 besitzt die Kolbenstange 23 im Ausführungsbeispiel einen runden Querschnitt, dessen Außendurchmesser etwa dem größten Außendurchmesser der Kolbenstange 23 entspricht. Dadurch kann der Anschlag 89 nicht in das Innengewinde 65 des Zustellteils 20 eingeschraubt werden. Auch eine andere Gestaltung des Anschlags 89, die ein Einschrauben in das Innengewinde 65 verhindert, kann jedoch vorteilhaft sein. Der Anschlag 89 ist so angeordnet, dass der Anschlag 89 am Zustellteil 20 anschlägt, wenn die noch im Behälter vorhandene Menge an Injektionsflüssigkeit eingestellt ist. Dadurch kann der Bediener keine Dosis einstellen, die größer als die noch im Behälter vorhandene Restmenge an Injektionsflüssigkeit ist.

Fig. 27 zeigt die Anordnung des Zustellteils 20 in einer Zwischenstellung 74 von Bedienelement 6, Dosierorgan 16 und Zustellteil 20. Die Rasten 67 sind zu den Längsstegen 52 beabstandet. Die Feder 82 (Fig. 8) wirkt auf das Dosierorgan 16 in Richtung auf die Nullstellung 85 des Dosierorgans 16. Über die beim Einstellen der Injektionsdosis geschlossene Kupplung 14 und den Mitnehmer 13 wirkt die Feder 82 auch auf das Zustellteil 20. Das Zustellteil 20 wird in Richtung des in Fig. 27 gezeigten Pfeils 86 in Richtung auf die vorangegangene Raststellung der Rasten 67 belastet. Sobald der Bediener die Stellhülse 7 loslässt, beispielsweise, um den Bedienknopf 8 zu drücken und eine Dosis zu injizieren, werden das Dosierorgan 16 und das Zustellteil 20 aufgrund der Kraft der Feder 82 bis zur vorangegangenen vorgesehenen Stellung, die der nächstkleineren vorgesehenen Dosis oder keiner Dosis von Injektionsflüssigkeit zugeordnet ist, zurückgestellt. Dabei dreht sich das Zustellteil 20 in Richtung des Pfeils 86. Vorgesehene Stellungen sind dabei Injektionsstellungen 73 oder die Nullstellung 85. Da keine nicht vorgesehene Menge an Injektionsflüssigkeit eingestellt werden kann, ist das Auspressen einer nicht vorgesehenen Menge an Injektionsflüssigkeit verhindert.

Wird das Bedienelement 6 und damit auch das Zustellteil 20 weiter gedreht, so gelangen die Rasten 67 nach Überwinden der Längsstege 52, die mit den Rasten 67 die Rasteinrichtung 26 bilden, in Drehrichtung 87 hinter die Längsstege 52. Die Drehrichtung 87 ist die Drehrichtung, in der sich das Zustellteil 20 und das Dosierorgan 16 beim Einstellen einer Dosis von Injektionsflüssigkeit drehen. Die Längsstege bilden an ihren in Richtung des Pfeils 86 vorne liegenden Seiten Rastelemente 53, an denen die Rasten 67 einrasten. Liegen die Rasten 67 an den Rastelementen 53 der Längsstege 52 an, so ist die Rasteinrichtung 26 eingerastet, und die Anordnung befindet sich in einer vorgesehenen Injektionsstellung 73. Eine weitere selbsttätige Bewegung des Zustellteils 20 in Richtung des Pfeils 86 aufgrund der Kraft der Feder 82 ist durch die Anlage der Rasten 67 an den Rastelementen 53 vermieden. Der Bediener kann den Bedienknopf 8 drücken und die eingestellte Dosis injizieren. Das Auspressen der eingestellten Menge an Injektionsflüssigkeit wird durch die Feder 82 unterstützt. An den Längsstegen 52 sind die Rasten 67 dabei parallel zur Längsmittelachse 50 des Injektionsgeräts 1 geführt. Die Längsstege 52 stellen dadurch sicher, dass das Zustellteil 20 sich beim Injizieren einer Dosis nicht um die Längsmittelachse 50 drehen und dadurch die auszupressende Menge an Injektionsflüssigkeit verringern kann.

Die Längsstege 52 besitzen an ihren in Drehrichtung 87 vorne liegenden Seiten jeweils eine Schräge 88, die die Rasten 67 radial nach innen auslenken und so das Überwinden der Längsstege 52 erleichtern. Die Schrägen 88 üben auf die Rasten 67 und damit auf das Zustellteil 20 und das Dosierorgan 16 eine der Drehrichtung 87 entgegengerichtete Kraft aus. In Stellungen des Dosierorgans 16, in denen die Rasten 67 an den Schrägen 88 anliegen, wird das Zustellteil 20 aufgrund der von der Rasteinrichtung 26 ausgeübten Kraft entgegen der Drehrichtung 87 zurückgestellt, bis die Rasten 67 nicht mehr an den Schrägen 88 anliegen, wenn der Bediener keine entgegengerichtete Kraft auf das Zustellteil 20 ausübt. In dem Winkelabstand β zwischen den Längsstegen 52 besitzen die Rasten 67 zum proximalen Teil 46 des Innenrohrs 17 einen Abstand und sind nicht in Kontakt mit dem proximalen Teil 46. Vorteilhaft verläuft der Innenumfang des proximalen Teils 46 in diesem Bereich in einem Kreisbogen um die Längsmittelachse 50. In diesem Bereich übt die Rasteinrichtung 26 keine Kraft auf das Zustellteil 20 oder das Dosierorgan 16 aus. Die Rückstellung des Zustellteils 20 und des Dosierorgans 16 in eine Injektionsstellung 73 oder die Nullstellung 85 erfolgt in diesem Bereich ausschließlich aufgrund der Kraft der Feder 82. Im Ausführungsbeispiel ist ein Injektionsgerät 1 gezeigt, bei dem nur eine einzige vorgegebene Menge an Injektionsflüssigkeit aus dem Behälter ausgepresst werden kann. Diese Menge ist erreicht, wenn das Bedienelement um 180° gedreht wurde. Es kann jedoch auch vorgesehen sein, dass mehrere Injektionsstellungen 73 möglich sind, die unterschiedlichen Mengen an Injektionsflüssigkeit zugeordnet sind.

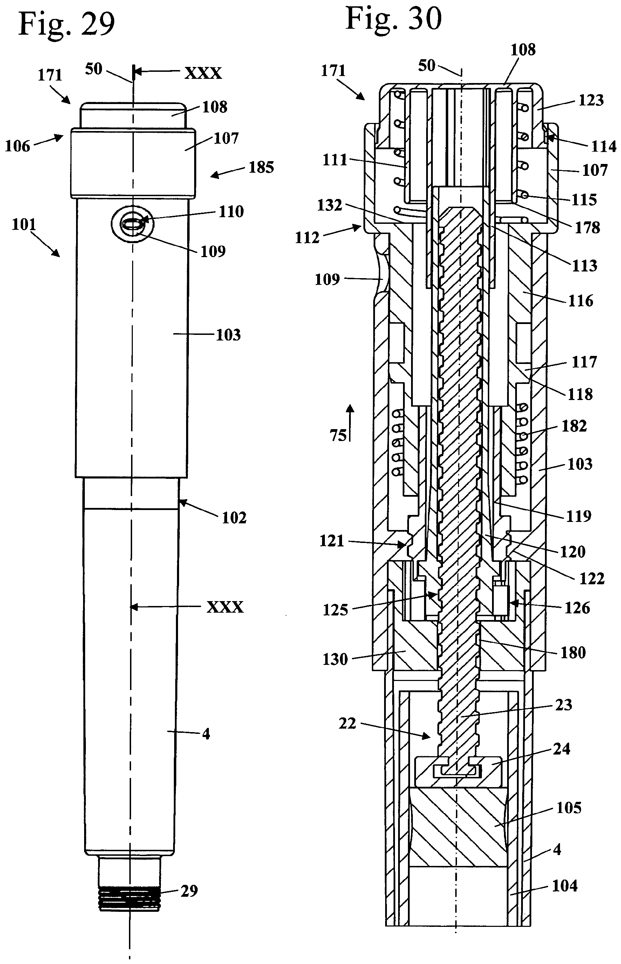

Die Figuren 29 bis 70 zeigen ein Ausführungsbeispiel eines Injektionsgeräts 101. Das Injektionsgerät 101 besitzt ein Gehäuse 102 mit einem oberen Gehäuseteil 103, an dem ein Halter 4 festgelegt ist. Gleiche Bezugszeichen wie in den vorangegangenen Figuren kennzeichnen dabei gleiche Elemente. Am distalen Ende des oberen Gehäuseteils 103 ist ein Bedienelement 106 angeordnet, das eine Stellhülse 107 und einen Betätigungsknopf 108 umfasst. In Fig. 29 befindet sich das Inj ektionsgerät 101 in seiner Nullstellung 185. Das obere Gehäuseteil 103 besitzt ein Sichtfenster 109, durch das eine Skala 110 sichtbar ist. In der Nullstellung 185 zeigt die Skala im Ausführungsbeispiel eine "0". Der Betätigungsknopf 108 befindet sich in Fig. 29 in seiner distalen Position 171.

Fig. 30 zeigt den Aufbau des Injektionsgeräts 101 im Einzelnen. Fig. 30 zeigt auch den im Halter 4 eingesetzten Behälter 104, an dessen Stopfen 105 die Kolbenscheibe 24 des Dosierkolbens 22 anliegt. Das Injektionsgerät 101 besitzt einen Kolbenstangenring 130, in dem die Kolbenstange 23 drehfest geführt ist. Über den Kolbenstangenring 130 ist die Kolbenstange 23 gegenüber dem Gehäuse 102 drehfest gehalten. Die Kolbenstange 23 ragt durch eine Öffnung 180 des Kolbenstangenrings 130. Das gezeigte Injektionsgerät 101 ist in einer Ausführung gezeigt, bei der der Anwender das Gerät mit dem schon eingesetzten Behälter 104 erhält, und bei der der Anwender das Injektionsgerät 101 zusammen mit dem Behälter 104 entsorgt, wenn der Behälter 104 leer ist. Ein Ersetzen des Behälters 104 ist nicht vorgesehen. Der Halter 4 ist fest mit dem oberen Gehäuseteil 103 verbunden, beispielsweise mit diesem unlösbar verschnappt oder eingeklebt. Der Kolbenstangenring 130 wird vom Halter 4 im oberen Gehäuseteil 103 in axialer Richtung, also in Richtung der Längsmittelachse 50, gesichert. Der Kolbenstangenring 130 ist außerdem drehfest im Gehäuse 103 gehalten, wie im Folgenden noch näher beschrieben wird.

Das Injektionsgerät 101 besitzt ein Zustellteil 120, das mit der Kolbenstange 23 über eine erste Gewindeverbindung 125 verbunden ist. Am proximalen Ende des Zustellteils 120 ist eine Rasteinrichtung 126 angeordnet. Die Rasteinrichtung 126 ist zwischen dem Kolbenstangenring 130 und dem Zustellteil 120 gebildet. Das Zustellteil 120 ist drehfest mit einem Mitnehmer 113 verbunden. Der Mitnehmer 113 ist drehfest mit dem Betätigungsknopf 108 verbunden. Im Ausführungsbeispiel ist der Mitnehmer 113 am Betätigungsknopf 108 angeformt, also einteilig mit dem Betätigungsknopf 108 ausgebildet.

Am Zustellteil 120 liegt ein Schieber 119 an, der in proximaler Richtung auf das Zustellteil 120 wirkt. Der Schieber 119 ist über eine zweite Gewindeverbindung 121 mit dem oberen Gehäuseteil 103 verbunden. Die zweite Gewindeverbindung 121 ist an einem Rand 122 des oberen Gehäuseteils 103 ausgebildet, der nach innen ragt. Der Schieber 119 ist drehfest mit einem Dosierorgan 116 verbunden. Das Dosierorgan 116 ist einteilig mit der Stellhülse 107 ausgebildet und bildet mit dieser ein Einstellteil 112. Das Einstellteil 112 ist drehbar aber axial fest im Gehäuse 102 gelagert. Hierzu besitzt das Einstellteil 112 am Dosierorgan 116 einen radial nach außen ragenden Rand 117, der am Gehäuse 102 einrastet, wie im Folgenden noch näher beschrieben wird. An seiner proximalen Seite besitzt der Rand 117 eine Fase 118 zur Erleichterung der Montage. Auf das Dosierorgan 116 ist die Skala 110 (Fig. 29) aufgebracht. Beim Einstellen einer auszupressenden Menge an Injektionsflüssigkeit kann die Stellhülse 107 um weniger als eine Umdrehung gedreht werden, so dass jedem Wert der Skala 110 eine eindeutige Menge an Injektionsflüssigkeit zugeordnet ist. Im Ausführungsbeispiel ist die Stellhülse 107 um eine halbe Umdrehung drehbar.

Die Stellhülse 107 ist an ihrem distalen Ende offen. In der Stellhülse 107 ist der Betätigungsknopf 108 axial verschiebbar gehalten. Der Betätigungsknopf 108 besitzt einen Rand 123, der in die Stellhülse 107 ragt. Zwischen dem Rand 123 und der Stellhülse 107 ist eine Kupplung 114 ausgebildet. In der in den Figuren 29 und 30 gezeigten distalen Position 171 des Betätigungsknopfs 108 ist die Kupplung 114 geschlossen und verbindet den Betätigungsknopf 108 drehfest mit der Stellhülse 107. Der Betätigungsknopf 108 besitzt einen zylindrischen Stutzen 111, der in radialer Richtung zwischen dem Mitnehmer 113 und dem Rand 123 angeordnet ist. Die Stirnseite des Stutzens 111 bildet einen Anschlag 178, der mit einer Schulter 132 des Einstellteils 112 zusammenwirkt und mit dieser die proximale Position des Betätigungsknopfs 108 festlegt. An der Außenseite des Stutzens 111 ist eine Druckfeder 115 angeordnet, die den Betätigungsknopf 108 in seine distale Position 171 vorspannt.

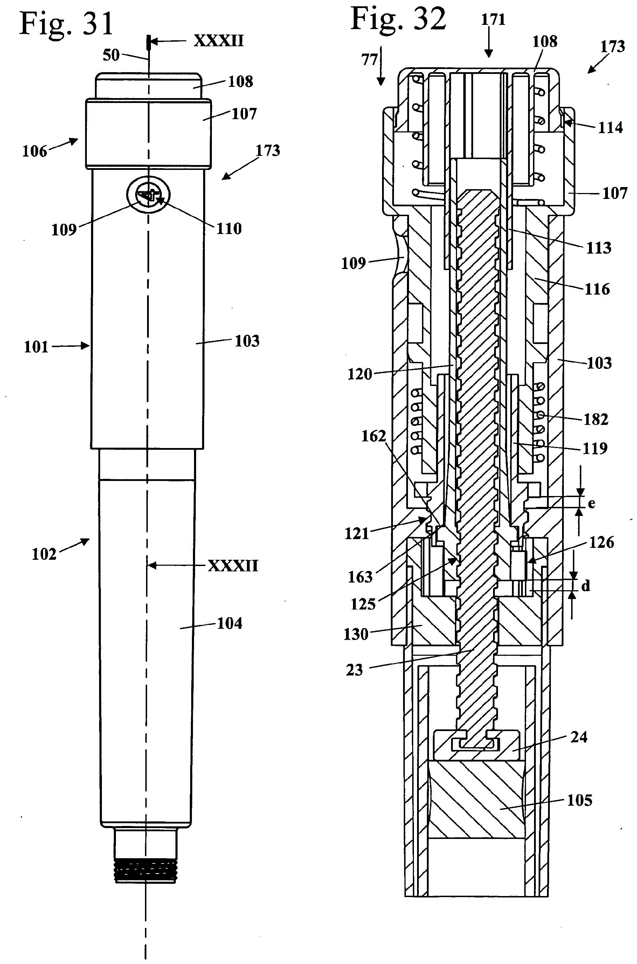

Die Figuren 31 und 32 zeigen das Injektionsgerät 101 nach dem Einstellen der Maximaldosis. Im Sichtfenster 109 zeigt die Skala 110 die Ziffer "4" an. In dieser Stellung befindet sich das Injektionsgerät 101 in einer Injektionsstellung 173, in der eine vorgesehene Menge an Injektionsflüssigkeit eingestellt ist. Wie Fig. 32 zeigt, ist die Kupplung 114 weiterhin geschlossen. Der Betätigungsknopf 108 befindet sich in seiner distalen Position 171. Um die auszupressende Menge an Injektionsflüssigkeit einzustellen, wurde die Stellhülse 107 aus der in den Figuren 29 und 30 gezeigten Stellung um eine halbe Umdrehung im Uhrzeigersinn gegenüber dem Gehäuse 102 gedreht. Dabei hat der Mitnehmer 113 das Zustellteil 120 gedreht. Aufgrund der ersten Gewindeverbindung 125 hat sich das Zustellteil 120 in distaler Richtung bewegt, und zwar um einen ersten Stellweg d. Das Dosierorgan 116 hat den Schieber 119 mitgenommen und gedreht. Über die zweite Gewindeverbindung 121 hat sich der Schieber 119 um einen zweiten Stellweg e in distale Richtung bewegt, der vorteilhaft mindestens dem ersten Stellweg d entspricht. Beim Drehen der Stellhülse 107 dreht sich das Zustellteil 120 gegenüber dem Gehäuseteil 103, wodurch die Rasteinrichtung 126 spür- und hörbare Klicks erzeugt.

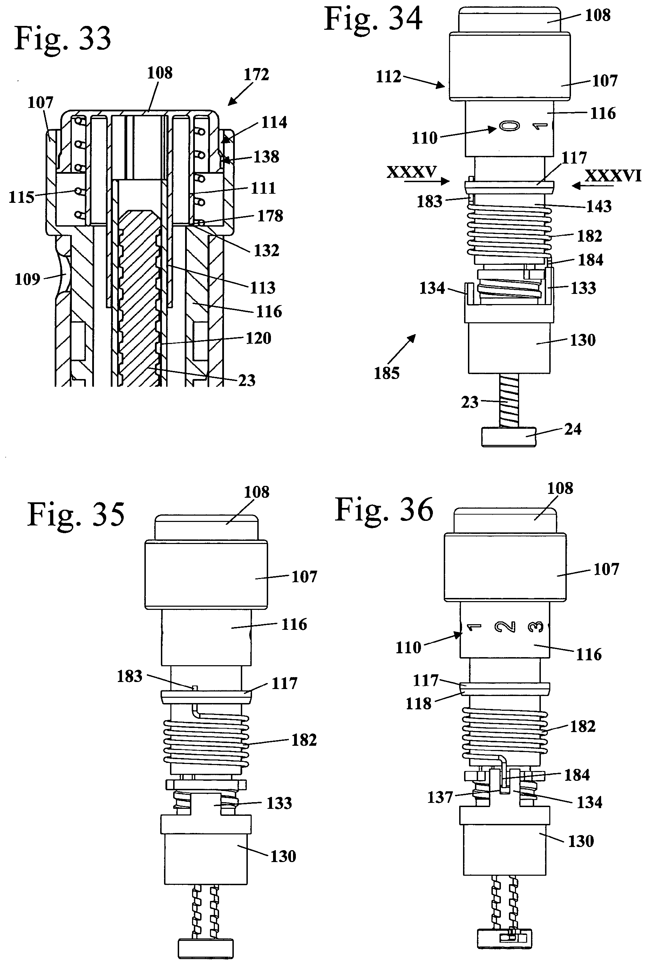

Zum Auspressen der eingestellten Menge an Injektionsflüssigkeit muss der Betätigungsknopf 108 in Richtung des Pfeils 77 in proximaler Richtung bewegt werden. Die proximale Position 172 des Betätigungsknopfs 108 ist in Fig. 33 gezeigt. In dieser Stellung ist die Kupplung 114 gelöst. Der Betätigungskopf 108 besitzt eine Außenverzahnung 138, die Teil der Kupplung 114 ist. Die Außenverzahnung 138 am Betätigungsknopf 108 hat sich bei der Bewegung des Betätigungsknopfs 108 in proximale Richtung aus dem Bereich der Gegenverzahnung an der Stellhülse 107 bewegt. Dadurch ist die Stellhülse 107 gegenüber dem Betätigungsknopf 108 drehbar. Die Kupplung 114 ist gelöst. Der Betätigungsknopf 108 kann in die Stellhülse 107 gedrückt werden, bis der Anschlag 178 an der Schulter 132 des Dosierorgans 116 anliegt. Der Anschlag 178 ist so ausgebildet, dass die Reibung zwischen Dosierorgan 116 und Betätigungsknopf 108 gering ist. Im Ausführungsbeispiel ist hierzu eine gerundete Kontur am Stutzen 111 vorgesehen.

Wie die Figuren 34 bis 36 zeigen, wirkt zwischen dem Dosierorgan 116 und dem gehäusefest gehaltenen Kolbenstangenring 130 eine Feder 182, die als Torsionsfeder ausgebildet ist. In der in den Figuren 34 bis 36 gezeigten Nullstellung 185 ist die Feder 182 vorzugsweise bereits vorgespannt. Dadurch wird sichergestellt, dass die Feder 182 die Injektion bis zum Ende in Gang halten, also den Stopfen 105 im Behälter 104 in die gewünschte Endposition schieben kann. Wird die Stellhülse 107 gegenüber dem Kolbenstangenring 130 im Uhrzeigersinn gedreht, so wird die Feder 182 weiter gespannt. Die Rasteinrichtung 126 verhindert nach jeder überwundenen Raste ein Zurückdrehen der Stellhülse 107 in die Nullstellung 185. Sobald die gewünschte Dosis eingestellt ist, ist der Betätigungsknopf 108 zu drücken. Dadurch löst sich die Kupplung 114 und die Stellhülse 107 wird von der Feder 182 zurück in ihre Nullstellung 185 gedreht. Die Drehbewegung des Einstellteils 112 mit dem Dosierorgan 116 bewirkt eine Drehung des Schiebers 119 gegenüber dem Gehäuseteil 103 und damit über die zweite Gewindeverbindung 121 eine axiale Bewegung des Schiebers 119 in proximaler Richtung, und zwar um den Stellweg e. Wie Fig. 32 zeigt, besitzt der Schieber 119 einen Mitnahmeabsatz 162, der mit einem Mitnahmeabsatz 163 des Zustellteils 120 zusammenwirkt und das Zustellteil 120 in proximaler Richtung mitnimmt. Dabei verschiebt der Schieber 119 das Zustellteil 120 um den ersten Stellweg d. Das Zustellteil 120 stützt sich dabei über die Rasteinrichtung 126 in Umfangsrichtung ab, so dass sich das Zustellteil 120 nicht drehen kann. Da die Kolbenstange 23 in der Kolbenführung 130 drehfest gehalten ist, wird die Kolbenstange 23 in proximaler Richtung bewegt und presst über den Stopfen 105 die eingestellte Menge an Injektionsflüssigkeit aus dem Behälter 104 aus.

Wie die Figuren 34 bis 36 zeigen, ist ein erstes Ende 183 der Feder 182 in den Rand 117 des Dosierorgans 116 eingehängt. Ein zweites Ende 184 der Feder 182 ist am Kolbenstangenring 130 gehalten. Wie die Figuren zeigen, besitzt der Kolbenstangenring 130 zwei Arme 133 und 134, die sich an gegenüberliegenden Seiten des Kolbenstangenrings 130 in distale, axiale Richtung erstrecken. Der erste Arm 133 besitzt einen Schlitz 137, in dem das zweite Ende 184 der Feder 182 geführt ist. Über den Kolbenstangenring 130 ist das zweite Ende 184 der Feder 182 drehfest mit dem Gehäuse 102 verbunden. Beim Einstellen einer Dosis wird die Feder 182 gespannt, da das erste Ende 183 gegenüber dem gehäusefest gehaltenen zweiten Ende 184 gedreht wird. Nach dem Einstellen einer Dosis wird die Anordnung über die Rasteinrichtung 126 in einer Injektionsstellung 173 gehalten, bis der Anwender durch Drücken des Betätigungsknopfs 108 die Kupplung 114 löst. Nach dem Lösen der Kupplung 114 dreht die Feder 182 das Einstellteil 112 zurück in die Nullstellung 185. Damit schraubt sich auch der Schieber 119 in die Nullstellung 185 und schiebt dabei das Zustellteil 120 und damit die Kolbenstange 23 in proximale Richtung, so dass die eingestellte Menge an Injektionsflüssigkeit aus dem Behälter 104 ausgepresst wird.

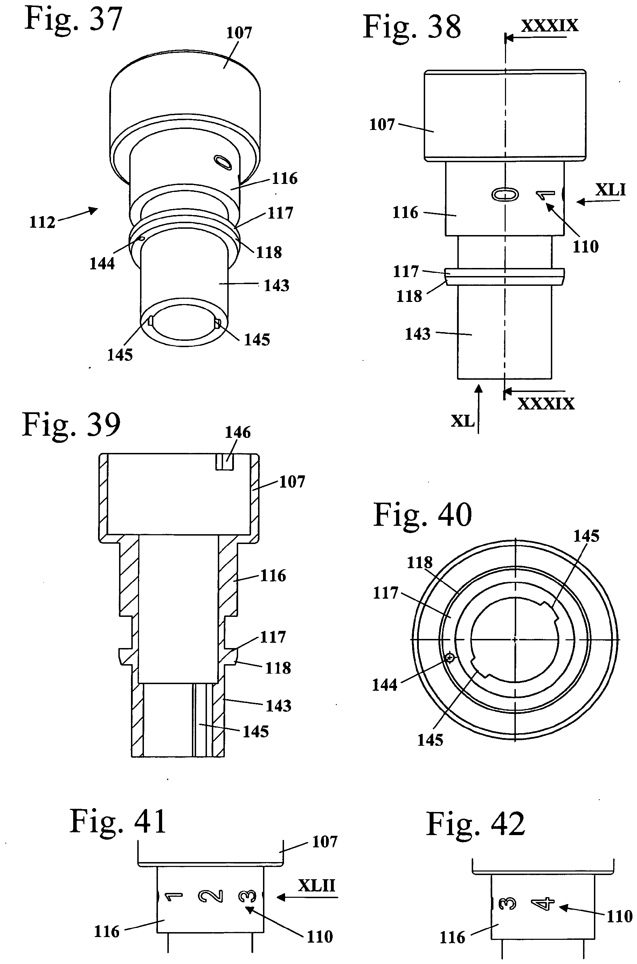

Die Figuren 37 bis 42 zeigen die Gestaltung des Einstellteils 112 im Einzelnen. Das Einstellteil 112 ist im Wesentlichen zylindrisch ausgebildet und besitzt einen Abschnitt mit vergrößertem Außendurchmesser, der die Stellhülse 107 bildet, sowie einen daran anschließenden Bereich mit verkleinertem Außendurchmesser, der das Dosierorgan 116 bildet. An den Rand 117 schließt ein Stutzen 143 an, der Führungsnuten 145 zur drehfesten Verbindung mit dem Schieber 119 besitzt. Im Ausführungsbeispiel sind zwei einander gegenüberliegende Führungsnuten 145 vorgesehen. Auch eine andere Anzahl und Anordnung von Führungsnuten 145 kann jedoch zweckmäßig sein. Am Außenumfang des Stutzens 143 ist die Feder 182 geführt, wie Fig. 34 zeigt. Wie Fig. 40 zeigt, besitzt der Rand 117 eine Öffnung 144, in der das erste Ende 183 der Feder 182 eingehängt ist.

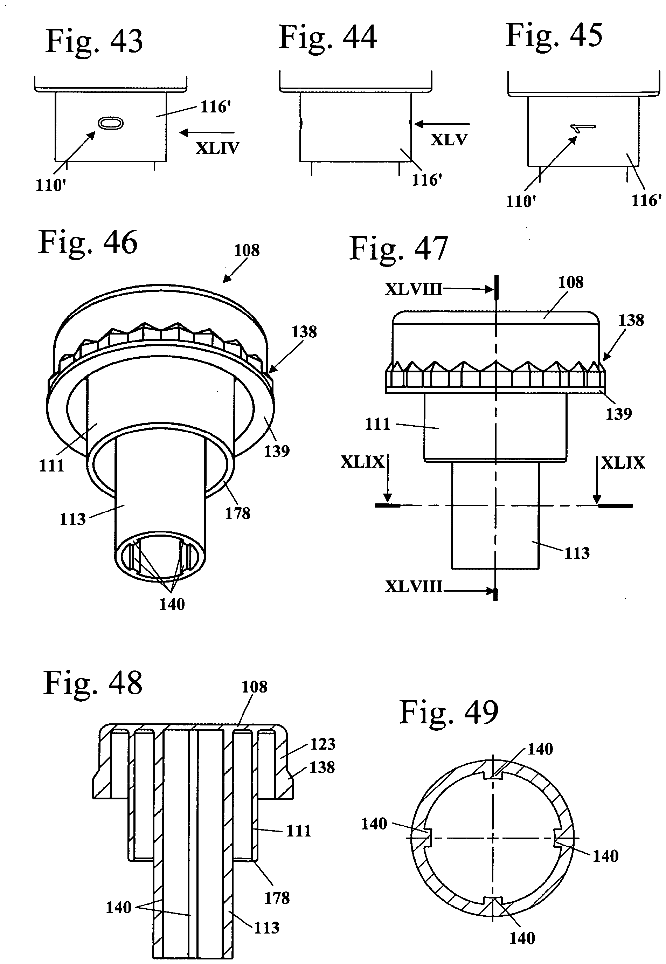

Wie Fig. 39 zeigt, ist an der Innenseite der Stellhülse 107 ein einziger Rastzahn 146 angeordnet, der mit der Außenverzahnung 138 des Betätigungsknopfs 108 zusammenwirkt. Auch eine größere Anzahl von Rastzähnen 146 kann jedoch vorgesehen sein. Dadurch, dass nur ein einziger Rastzahn 146 vorgesehen ist, kann der Betätigungsknopf 108 einfach durch axiales Einschieben und Verrasten hinter dem Rastzahn 146 in der Stellhülse 107 montiert werden. Wie Fig. 46 zeigt, besitzt der Betätigungsknopf 108 an der distalen Seite der Außenverzahnung 138 einen Rand 139, der hinter dem Rastzahn 146 verrastet. Dadurch ist der Betätigungsknopf 108 in axialer Richtung in der Stellhülse 107 gesichert. Die Figuren 38, 41 und 42 zeigen die Skala 110. Die Skala 110 zeigt eine mit "0" bezeichnete Nullstellung 185 sowie vier mit "1 ", "2", "3", und "4" bezeichnete Injektionsstellungen 173 an. Die Injektionsstellungen 173 sind in gleichem Abstand zueinander angeordnet.

Die Figuren 43 bis 45 zeigen ausschnittsweise ein Ausführungsbeispiel eines Dosierorgans 116'. Das Dosierorgan 116' besitzt eine Skala 110' mit einer mit "0" bezeichneten Nullstellung 185 und einer einzigen Injektionsstellung 173, die mit "1" gekennzeichnet ist. Bei einem Injektionsgerät 101 mit dem Dosierorgan 116' kann nur eine einzige, konstruktiv vorgegebene Menge an auszupressender Injektionsflüssigkeit eingestellt werden.

Wie die Figuren 46 bis 49 zeigen, besitzt der Mitnehmer 113 an seiner Innenseite Führungsstege 140, die zur drehfesten Verbindung mit dem Zustellteil 120 dienen. Im Ausführungsbeispiel sind vier gleichmäßig am Innenumfang verteilte Führungsstege 140 vorgesehen. Wie insbesondere Fig. 48 zeigt, ist der Rand 123 in axialer Richtung kürzer als der Stutzen 111, und der Mitnehmer 113, der innerhalb des Stutzens 111 angeordnet ist, ist länger als der Stutzen 111.

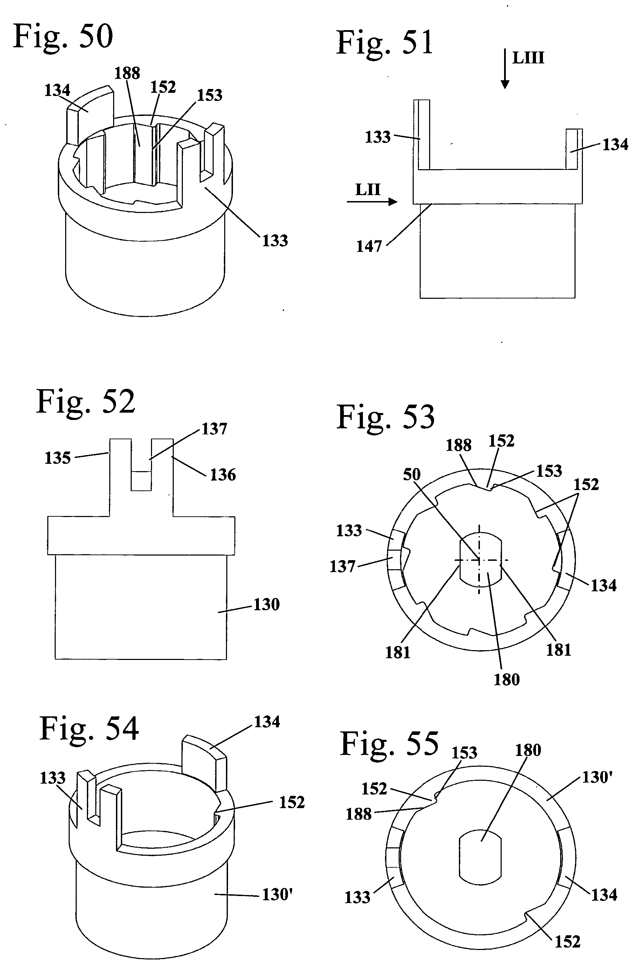

Die Figuren 50 bis 53 zeigen den Kolbenstangenring 130. Der Kolbenstangenring 130 besitzt einen Absatz 147, an dem in montiertem Zustand der Halter 4 anliegt und den Kolbenstangenring 130 im Gehäuse 102 in axialer Richtung fixiert. Wie die Figuren 50 und 53 zeigen, besitzt der Kolbenstangenring 130 an seinem Innenumfang eine Verzahnung, die durch mehrere Längsstege 152 gebildet ist. Die Längsstege 152 besitzen an einer Seite jeweils ein Rastelement 153 und an der gegenüberliegenden Seite eine Schräge 188. Die Längsstege 152 sind asymmetrisch ausgebildet. Die Rastelemente 153 sind näherungsweise in radialer Richtung zur Längsmittelachse 50 ausgerichtet. Dadurch ist ein Zurückdrehen einer einmal eingestellten Injektionsstellung in die nächst niedrige Injektionsstellung nicht möglich. Die Rastelemente 153 können jedoch auch so gestaltet werden, dass ein Zurückdrehen möglich ist. Die Rastelemente 153 sind so zu gestalten, dass die Rastelemente 153 nicht allein aufgrund des von der Feder 182 aufgebrachten Drehmoments überwunden werden können. Im Ausführungsbeispiel sind acht Rastelemente 153 gleichmäßig am Innenumfang des Kolbenstangenrings 130 verteilt angeordnet. Auch eine andere Anzahl von Rastelementen 153 kann vorteilhaft sein, um gewünschte vorgegebene Mengen an Injektionsflüssigkeit einstellen zu können.

Zwischen den Längsstegen 152 ist jeweils ein Bereich angeordnet, in dem die Innenwand des Kolbenstangenrings 130 kreisbogenförmig um die Längsmittelachse 50 verläuft. Wie Fig. 53 zeigt, besitzt der Kolbenstangenring 130 die Öffnung 180 zur drehfesten Fixierung der Kolbenstange 23. Zur drehfesten Fixierung besitzt die Öffnung 180 Seitenwände 181, die gerade verlaufen. Wie die Figuren 51 und 52 zeigen, ist der zweite Arm 134 in axialer Richtung deutlich kürzer als der erste Arm 133 ausgebildet. Am ersten Arm 133 sind Anschläge 135 und 136 gebildet, mit denen der Schieber 119 zusammenwirkt. Der zweite Arm 134 ragt nicht bis in den Bereich der Anschläge des Schiebers 119.

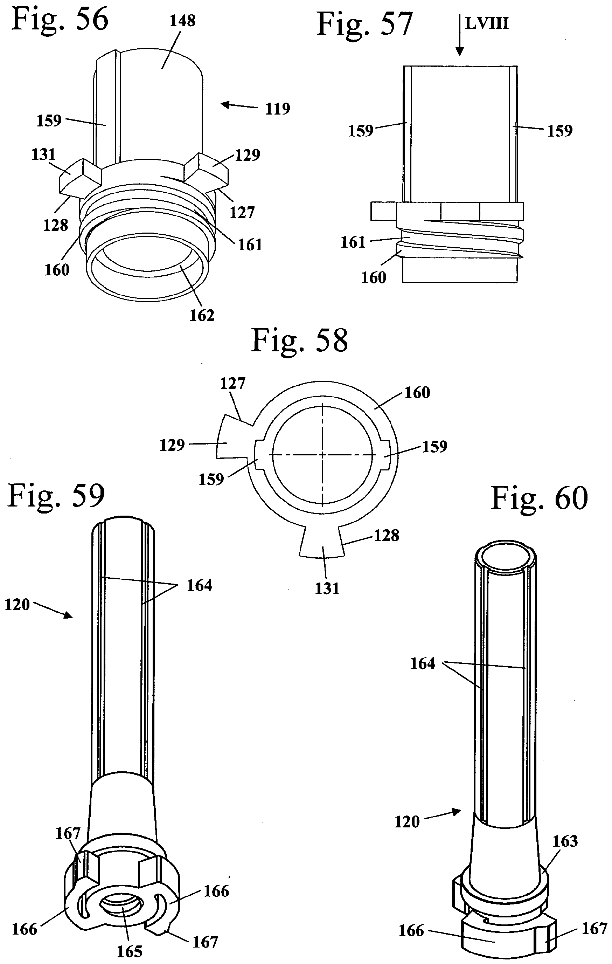

Die Figuren 54 und 55 zeigen die Gestaltung des Kolbenstangenrings 130' für ein Injektionsgerät 101, bei dem nur eine einzige Dosis eingestellt werden kann und das das Dosierorgan 116' aus den Figuren 43 bis 45 besitzen kann. Wie die Figuren 54 und 55 zeigen, sind zwei Längsstege 152 einander gegenüberliegend angeordnet. An den Längsstegen 152 sind die Rastelemente 153 und die Schrägen 188 ausgebildet. In der einzigen Injektionsstellung 173 rastet an jedem Rastelement 153 eine Raste 167 (Fig. 59) ein.

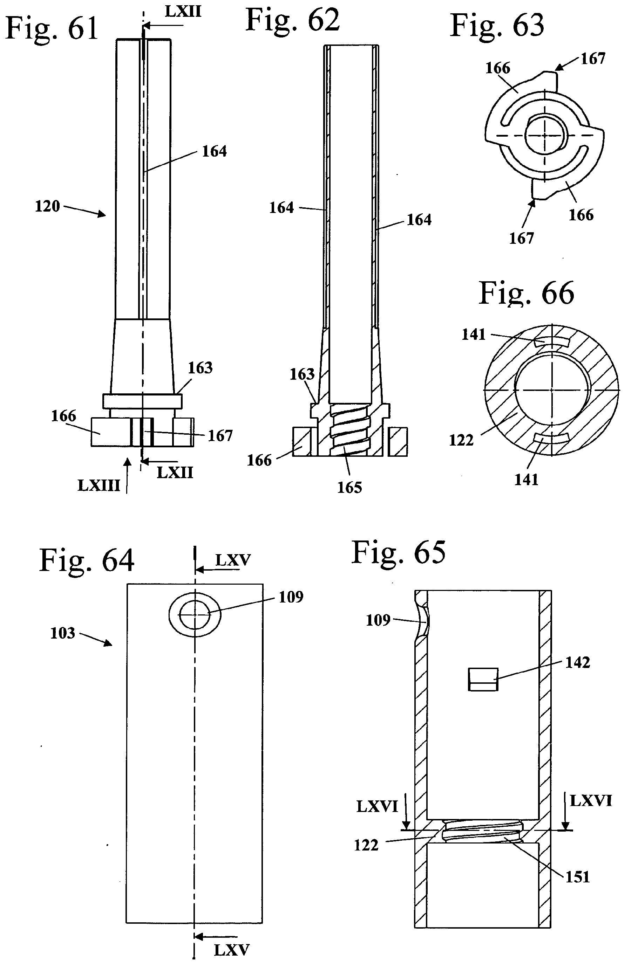

Die Figuren 56 bis 58 zeigen den Schieber 119 im Einzelnen. Der Schieber 119 besitzt einen zylindrischen Abschnitt 148, der an seiner Außenseite Längsstege 159 aufweist, die in die Führungsnuten 145 des Dosierorgans 116 ragen. Dadurch ist der Schieber 119 mit dem Dosierorgan 116 drehfest verbunden. Der Schieber 119 besitzt an seiner distalen Seite einen Ringsteg 160, der ein Außengewinde 161 aufweist. Das Außengewinde 161 bildet mit dem in Fig. 65 gezeigten Innengewinde 151 des oberen Gehäuseteils 103 die zweite Gewindeverbindung 121. Zwischen dem Ringsteg 160 und dem zylindrischen Abschnitt 148, der die Längsstege 159 trägt, sind zwei Arme 129 und 131 angeordnet, die radial nach außen ragen. An dem ersten Arm 129 ist ein erster Anschlag 127 ausgebildet, der mit dem ersten Anschlag 135 des ersten Arms 133 des Kolbenstangenrings 130 zusammenwirkt und mit diesem die Nullstellung des Injektionsgeräts 101 definiert. Der zweite Arm 131 besitzt einen zweiten Anschlag 128, der mit dem zweiten Anschlag 136 des Kolbenstangenrings 130 die Maximaldosis, also die maximal einzustellende Menge an auszupressender Injektionsflüssigkeit, definiert. Zwischen der Nullstellung und der Maximaldosis ist der Schieber 119 gegenüber dem Kolbenstangenring 130 im Ausführungsbeispiel um eine halbe Umdrehung drehbar. Auch andere Drehbereiche können vorteilhaft sein.

Die Figuren 59 bis 63 zeigen das Zustellteil 120 im Einzelnen. Das Zustellteil 120 besitzt einen hülsenförmigen Abschnitt, der an seiner Außenseite Längsnuten 164 aufweist. Im Ausführungsbeispiel sind vier Längsnuten 164 vorgesehen. In die Längsnuten 164 ragen die Führungsstege 140 des Betätigungsknopfs 108. Dadurch sind Betätigungsknopf 108 und Zustellteil 120 drehfest miteinander verbunden. An seiner proximalen Seite weist das Zustellteil 120 zwei Rastarme 166 auf, die an ihren freien Enden jeweils eine Raste 167 tragen. Das Zustellteil 120 besitzt außerdem an seinem proximalen Ende ein Innengewinde 165, in das die Kolbenstange 23 eingeschraubt ist und mit diesem die zweite Gewindeverbindung 121 bildet. In den Figuren 61 und 62 ist auch der Mitnahmeabsatz 163 gezeigt, an dem der Mitnahmeabsatz 162 des Schiebers 119 anliegt.

Die Figuren 64 bis 66 zeigen das obere Gehäuseteil 103. Das obere Gehäuseteil 103 ist hülsenförmig ausgebildet und besitzt benachbart zu seinem distalen Ende das Sichtfenster 109. Der nach innen ragende Rand 122 weist das Innengewinde 151 auf. Das obere Gehäuseteil 103 besitzt an seiner Innenseite eine Rasterhöhung 142, hinter der der Rand 117 des Einstellteils 112 (Figuren 37 bis 40) einrastet.

Wie Fig. 66 zeigt, besitzt der Rand 122 zwei einander gegenüberliegend angeordnete Öffnungen 141. Durch die Öffnungen 141 ragen die Arme 133 und 134 des Kolbenstangenrings 130 (Fig. 50). Dadurch ist der Kolbenstangenring 130 drehfest im Gehäuse 102 gehalten.

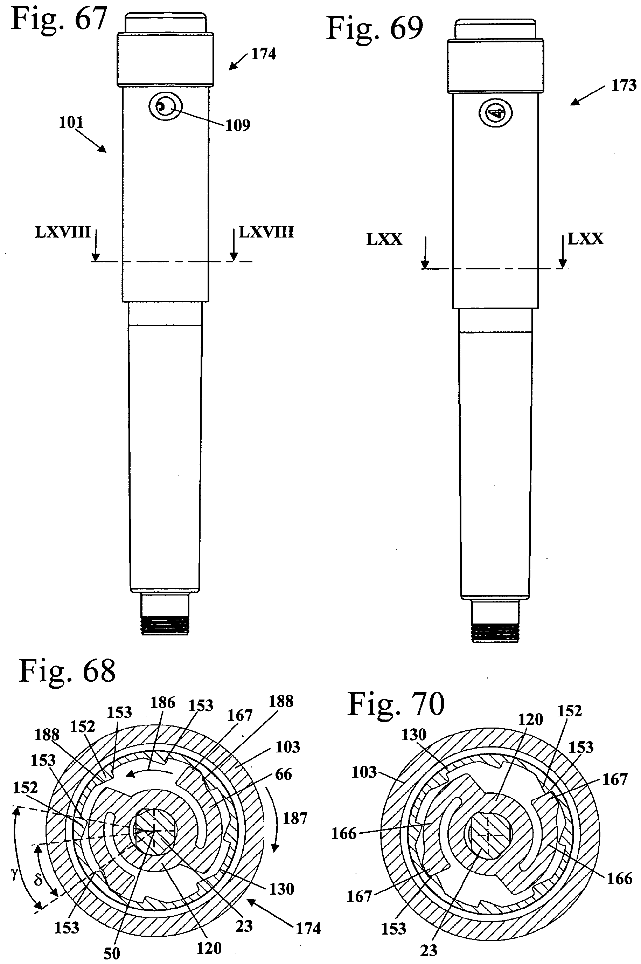

Fig. 67 zeigt das Injektionsgerät 101 in einer Zwischenstellung 174, in der eine nicht vorgesehene Menge an Injektionsflüssigkeit eingestellt ist. Im Sichtfenster 109 ist ein Teil der Ziffer "3" der Skala 110 erkennbar. Wie Fig. 68 zeigt, befinden sich die Rasten 167 in dieser Stellung benachbart zu Schrägen 188. Die Rasten 167 sind nicht an Rastelementen 153 eingerastet. Die Feder 182 übt in dieser Stellung eine Kraft auf das Zustellteil 120 in Richtung des Pfeils 186 aus und dreht das Zustellteil 120, bis die Rasten 167 an Rastelementen 153 anliegen. Die Rastelemente 153 sind in Umfangsrichtung um einen Winkel γ um die Längsmittelachse 50 voneinander beabstandet, der etwa 45° beträgt. Benachbarte Längsstege 152 besitzen zueinander einen Winkelabstand δ, der beispielsweise von etwa 20° bis etwa 40° betragen kann. Der Winkelabstand δ entspricht dem Winkelbereich, in dem das Dosierorgan 116 zwischen zwei Injektionsstellungen 173 oder einer Injektionsstellung 173 und einer Nullstellung 185 in Zwischenstellungen 174 gestellt werden kann. Eine Zwischenstellung 174 ist in Fig. 68 gezeigt. In Zwischenstellungen 174 bewegt die Torsionsfeder 182 das Zustellteil 120 in Richtung des Pfeils 186, sobald der Bediener die Stellhülse 107 loslässt, bis die nächst niedrige Injektionsstellung 173 oder die Nullstellung 185 erreicht ist. Um das Zustellteil 120 in eine Injektionsstellung 173 zu stellen, kann der Bediener das Zustellteil 120 auch in Richtung des Pfeils 187 weiterdrehen, bis die in den Figuren 69 und 70 gezeigte Injektionsstellung 173 erreicht ist. In dieser Stellung liegen die Rasten 167 hinter Rastelementen 153 an und sind entgegen der Federkraft (Pfeil 186 in Fig. 68) gehalten. Das Zustellteil 120 ist beim Auspressen einer eingestellten Menge an Injektionsflüssigkeit mit den Rasten 167 an den Längsstegen 152 in axialer Richtung gerührt.

Das Injektionsgerät 101 ist als Einmal-Injektionsgerät gezeigt, bei dem ein Austausch des Behälters 104 nicht möglich ist. Anstatt des Kolbenstangenrings 130 kann jedoch auch ein Kolbenstangenring 30 eingesetzt werden, der axial verschiebbar am oberen Gehäuseteil gehalten ist und einen Wechsel des Behälters 104 erlaubt.

## Patentansprüche

1. Injektionsgerät mit einem Gehäuse (2, 102), in dem eine Aufnahme (5) für einen Behälter mit Injektionsflüssigkeit ausgebildet ist, wobei das Injektionsgerät (1, 101) ein Bedienelement (6, 106) zur Einstellung einer Injektionsdosis besitzt, wobei das Injektionsgerät (1, 101) ein Dosierorgan (16, 116) besitzt, das beim Einstellen der Injektionsdosis gegenüber dem Gehäuse (2, 102) **um eine Längsmittelachse (50) des Injektionsgeräts (1, 101) gedreht** wird, wobei das Dosierorgan (16, 116) eine Nullstellung (85, 185) und mindestens eine Injektionsstellung (73, 173) besitzt, wobei in der Nullstellung (85, 185) keine Dosis eingestellt ist, und wobei in jeder Injektionsstellung (73, 173) eine vorgesehene Dosis an Injektionsflüssigkeit eingestellt ist, wobei das Injektionsgerät (1, 101) eine Rasteinrichtung (26, 126) besitzt, die zwischen **einem Zustellteil (20, 120) und dem Gehäuse (2, 102) wirkt, wobei das Zustellteil (20, 120) zur Einstellung einer Injektionsdosis um die Längsmittelachse (50) des Injektionsgeräts (1, 101) drehbar ist, wobei das Zustellteil (20, 120) beim Einstellen einer Injektionsdosis drehfest mit dem Dosierorgan (16,116) verbunden ist, wobei das Zustellteil (20, 120) sich beim Auspressen der Dosis in Richtung der Längsmittelachse (50) gegenüber dem Gehäuse (2, 102) bewegt und an mindestens einem Längssteg (52,152) des Gehäuses (2, 102) geführt ist, wobei an dem Längssteg (52, 152) mindestens ein Rastelement (53, 153) der Rasteinrichtung (26, 126) ausgebildet ist,** und wobei jeder Injektionsstellung (73, 173) des Dosierorgans (16, 116) eine Raststellung der Rasteinrichtung (26, 126) zugeordnet ist,
**dadurch gekennzeichnet, dass** das Dosierorgan (16, 116) in mindestens eine Zwischenstellung (74) stellbar ist, in der keine vorgesehene Dosis an Injektionsflüssigkeit eingestellt ist, und dass zwischen dem Dosierorgan (16, 116) und dem Gehäuse (2, 102) eine Feder (82, 182) wirkt, die das Dosierorgan (16, 116) bei unbetätigtem Bedienelement (6, 106) aus einer Zwischenstellung (74) in eine Injektionsstellung (73, 173) oder die Nullstellung (85, 185) zurückstellt.

2. Injektionsgerät nach Anspruch 1,
**dadurch gekennzeichnet, dass** die Feder (82, 182) beim Einstellen einer Injektionsdosis gespannt wird.

3. Injektionsgerät nach Anspruch 1 oder 2,
**dadurch gekennzeichnet, dass** die Feder (82, 182) zwischen dem Dosierorgan (16, 116) und dem Gehäuse (2, 102) wirkt.

4. Injektionsgerät nach einem der Ansprüche 1 bis 3,
**dadurch gekennzeichnet, dass** die Rasteinrichtung (26, 126) in Zwischenstellungen (74) des Dosierorgans (16, 116) keine Kraft auf das Dosierorgan (16, 116) ausübt.

5. Injektionsgerät nach Anspruch **einem der Ansprüche 1 bis 4,**
**dadurch gekennzeichnet, dass** mindestens zwei Injektionsstellungen (73, 173) vorgesehen sind, die in Umfangsrichtung um die Längsmittelachse (50) einen Winkelabstand (β, δ) von mindestens etwa 30° zueinander aufweisen.

6. Injektionsgerät nach einem der Ansprüche **1** bis **5**,
**dadurch gekennzeichnet, dass** das Bedienelement (6, 106) mehrteilig ausgebildet ist und einen Betätigungsknopf (8, 108) und eine Stellhülse (7, 107) besitzt, wobei die Stellhülse (7, 107) fest mit dem Dosierorgan (16, 116) verbunden ist, wobei der Betätigungsknopf (8, 108) über einen Mitnehmer (13, 113) mit dem Zustellteil (20, 120) verbunden ist, und wobei der Betätigungsknopf (8, 108) zum Auspressen von Injektionsflüssigkeit aus dem Behälter in Richtung der Längsmittelachse (50) in proximaler Richtung des Injektionsgeräts (1, 101) verschoben wird.

7. Injektionsgerät nach Anspruch **6**,
**dadurch gekennzeichnet, dass** der Betätigungsknopf (8, 108) über eine Kupplung (14, 114) mit der Stellhülse (7, 107) verbunden ist, wobei die Kupplung (14, 114) in einer ersten, distalen Position (71, 171) des Betätigungsknopfs (8, 108) eine drehfeste Verbindung zwischen dem Mitnehmer (13, 113) und der Stellhülse (7, 107) herstellt und in einer zweiten, proximalen Position (72) des Betätigungsknopfs (8, 108) eine Drehung der Stellhülse (7, 107) gegenüber dem Mitnehmer (13, 113) zulässt.

8. Injektionsgerät nach einem der Ansprüche **1** bis **7**,
**dadurch gekennzeichnet, dass** die Drehbewegung des Zustellteils (20, 120) beim Einstellen der Injektionsdosis über eine erste Gewindeverbindung (25, 125) eine axiale Bewegung des Zustellteils (20, 120) bewirkt, wobei das Zustellteil (20, 120) um einen ersten Stellweg (a, d) in Richtung der Längsmittelachse (50) des Injektionsgeräts (1, 101) verschoben wird.

9. Injektionsgerät nach Anspruch **8**,
**dadurch gekennzeichnet, dass** das Dosierorgan (116) drehbar und in Richtung der Längsmittelachse (50) unverschiebbar im Gehäuse (102) gelagert ist.

10. Injektionsgerät nach Anspruch **8** oder **9**,
**dadurch gekennzeichnet, dass** das Injektionsgerät (101) einen Schieber (119) besitzt, der ein Gewinde einer zweiten Gewindeverbindung (121) trägt, wobei die Drehbewegung des Schiebers (119) eine Bewegung in distaler Richtung der Längsmittelachse (50) um einen zweiten Stellweg (e) bewirkt, der mindestens so groß wie der erste Stellweg (d) ist, und dass der Schieber (119) einen Mitnahmeabsatz (162) besitzt, der mit einem Mitnahmeabsatz (163) des Zustellteils (120) zusammenwirkt und der eine axiale Bewegung des Schiebers (119) in proximaler Richtung auf das Zustellteil (120) überträgt.

11. Injektionsgerät nach Anspruch **10**,
**dadurch gekennzeichnet, dass** das Dosierorgan (16) über eine zweite Gewindeverbindung (18) mit dem Gehäuse (2) verbunden ist, wobei die Drehbewegung des Dosierorgans (16) eine Bewegung des Dosierorgans (16) und des Bedienelements (6) in Richtung der Längsmittelachse (50) des Injektionsgeräts (1) um einen zweiten Stellweg (b) bewirkt, wobei der zweite Stellweg (b) größer als der erste Stellweg (a) ist.

12. Injektionsgerät nach Anspruch **11**,
**dadurch gekennzeichnet, dass** das Injektionsgerät (1) einen Schieber (19) besitzt, der ein Gewinde einer dritten Gewindeverbindung (21) trägt, wobei die Drehbewegung des Schiebers (19) eine Bewegung in distaler Richtung der Längsmittelachse (50) um einen dritten Stellweg (c) bewirkt, der mindestens so groß wie der erste Stellweg (a) ist, und dass der Schieber (19) einen Mitnahmeabsatz (62) besitzt, der mit einem Mitnahmeabsatz (63) des Zustellteils (20) zusammenwirkt und der eine axiale Bewegung des Schiebers (19) in proximaler Richtung auf das Zustellteil (20) überträgt.

## Claims

1. Injection device having a housing (2, 102), in which a receptacle (5) for a container with injection liquid is formed, wherein the injection device (1, 101) has an operating element (6, 106) for setting an injection dose, wherein the injection device (1, 101) has a metering element (16, 116), which is turned about a central longitudinal axis (50) of the injection device (1, 101), when setting the injection dose, with respect to the housing (2, 102), wherein the metering element (16, 116) has a zero position (85, 185) and at least one injection position (73, 173), wherein in the zero position (85, 185) no dose is set, and wherein in each injection position (73, 173) a provided dose of injection liquid is set, wherein the injection device (1, 101) has a latching means (26, 126), which acts between a feed part (20, 120) and the housing (2, 102), wherein the feed part (20, 120) can be turned about the central longitudinal axis (50) of the injection device (1, 101) to set an injection dose, wherein the feed part (20, 120) is connected in a rotationally fixed manner to the metering element (16, 116) when setting an injection dose, wherein the feed part (20, 120) moves, when expressing the dose, in the direction of the central longitudinal axis (50) with respect to the housing (2, 102) and is guided on at least one longitudinal web (52, 152) of the housing (2, 102), wherein at least one latching element (53, 153) of the latching means (26, 126) is formed on the longitudinal web (52, 152), wherein a latching position of the latching means (26, 126) is assigned to each injection position (73, 173) of the metering element (16, 116), **characterised in that** the metering element (16, 116) can be brought into at least one intermediate position (74), in which no provided dose of injection liquid is set, and a spring (82, 182) acts between the metering element (16, 116) and the housing (2, 102), said spring resetting the metering element (16, 116), when the operating element (6, 106) is not actuated, from an intermediate position (74) into an injection position (73, 173) or the zero position (85, 185).

2. Injection device according to claim 1,
**characterised in that** the spring (82, 182) is tensioned when setting the injection dose.

3. Injection device according to claim 1 or 2,
**characterised in that** the spring (82, 182) acts between the metering element (16, 116) and the housing (2, 102).

4. Injection device according to one of claims 1 to 3,
**characterised in that** the latching means (26, 126) does not exert any force on the metering element (16, 116) in intermediate positions (74) of the metering element (16, 116).

5. Injection device according to one of claims 1 to 4,
**characterised in that** at least two injection positions (73, 173) are provided, which have an angular distance (β, δ) of at least approximately 30° relative to each other in the circumferential direction around the central longitudinal axis (50).

6. Injection device according to one of claims 1 to 5,
**characterised in that** the operating element (6, 106) is formed in multiple parts and has an actuating knob (8, 108) and an adjusting sleeve (7, 107), wherein the adjusting sleeve (7, 107) is fixedly connected to the metering element (16, 116), wherein the actuating knob (8, 108) is connected via an entrainer (13, 113) to the feed part (20, 120), and wherein the actuating knob (8, 108) is displaced, for the expression of injection liquid out of the container, in the direction of the central longitudinal axis (50) in the proximal direction of the injection device (1, 101).

7. Injection device according to claim 6,
**characterised in that** the actuating knob (8, 108) is connected via a coupling element (14, 114) to the adjusting sleeve (7, 107), wherein the coupling element (14, 114) produces, in a first, distal position (71, 171) of the actuating knob (8, 108), a rotationally fixed connection between the entrainer (13, 113) and the adjusting sleeve (7, 107), and allows, in a second, proximal position (72) of the actuating knob (8, 108), a rotation of the adjusting sleeve (7, 107) with respect to the entrainer (13, 113).

8. Injection device according to one of claims 1 to 7,
**characterised in that** the rotation movement of the feed part (20, 120), when setting the injection dose, causes an axial movement of the feed part (20, 120) via a first threaded connection (25, 125), wherein the feed part (20, 120) is displaced by a first travel range (a, d) in the direction of the central longitudinal axis (50) of the injection device (1, 101).

9. Injection device according to claim 8,
**characterised in that** the metering element (116) is mounted in the housing (102) rotatably and non-displaceably in the direction of the central longitudinal axis (50).

10. Injection device according to claim 8 or 9,
**characterised in that** the injection device (101) has a slide element (119), which bears a thread of a second threaded connection (121), wherein the rotation movement of the slide element (119) causes a movement in the distal direction of the central longitudinal axis (50) by a second travel range (e), which is at least as large as the first travel range (d), and the slide element (119) has an entrainment shoulder (162), which interacts with an entrainment shoulder (163) of the feed part (120) and which transfers an axial movement of the slide element (119) in the proximal direction to the feed part (120).

11. Injection device according to claim 10,
**characterised in that** the metering element (16) is connected to the housing (2) via a second threaded connection (18), wherein the rotation movement of the metering element (16) causes a movement of the metering element (16) and the operating element (6) in the direction of the central longitudinal axis (50) of the injection device (1) by a second travel range (b), wherein the second travel range (b) is larger than the first travel range (a).

12. Injection device according to claim 11,
**characterised in that** the injection device (1) has a slide element (19), which bears a thread of a third threaded connection (21), wherein the rotation movement of the slide element (19) causes a movement in the distal direction of the central longitudinal axis (50) by a third travel range (c), which is at least as large as the first travel range (a), and the slide element (19) has an entrainment shoulder (62), which interacts with an entrainment shoulder (63) of the feed part (20) and which transfers an axial movement of the slide element (19) in the proximal direction to the feed part (20).

## Revendications

1. Dispositif d'injection avec un boîtier (2, 102) dans lequel est formé un logement (5) pour un récipient avec un liquide d'injection, le dispositif d'injection (1, 101) comportant un élément de commande (6, 106) pour régler une dose d'injection, le dispositif d'injection (1, 101) comportant un organe de dosage (16, 116) qui, lors du réglage de la dose d'injection, est tourné sur un axe longitudinal médian (50) du dispositif d'injection (1, 101) par rapport au boîtier (2, 102), l'organe de dosage (16, 116) présentant une position zéro (85, 185) et au moins une position d'injection (73, 173), dans la position zéro (85, 185) aucune dose n'étant réglée, et dans chaque position d'injection (73, 173) une dose prévue de liquide d'injection étant réglée, le dispositif d'injection (1, 101) comportant un dispositif d'enclenchement (26, 126) qui agit entre un élément de positionnement (20, 120) et le boîtier (2, 102), l'élément de positionnement (20, 120), pour le réglage d'une dose d'injection, étant apte à tourner sur l'axe longitudinal médian (50) du dispositif d'injection (1, 101), l'élément de positionnement (20, 120) étant relié fixe en rotation à l'organe de dosage (16, 116) lors du réglage d'une dose d'injection, l'élément de positionnement (20, 120), lors de l'éjection de la dose, se déplaçant par rapport au boîtier (2, 102) dans le sens de l'axe longitudinal médian (50) et étant guidé sur au moins une nervure longitudinale (52, 152) du boîtier (2, 102), au moins un élément d'enclenchement (53, 153) du dispositif d'enclenchement (26, 126) étant formé sur la nervure longitudinale (52, 152), et une position d'enclenchement du dispositif d'enclenchement (26, 126) étant associée à chaque position d'injection (73, 173) de l'organe de dosage (16, 116), **caractérisé en ce que** l'organe de dosage (16, 116) est apte à être amené dans au moins une position intermédiaire (74) dans laquelle aucune dose prévue de liquide d'injection n'est réglée, et **en ce qu'**entre l'organe de dosage (16, 116) et le boîtier (2, 102) agit un ressort (82, 182) qui ramène l'organe de dosage (16, 116), quand l'élément de commande (6, 106) est non actionné, d'une position intermédiaire (74) jusqu'à une position d'injection (73, 173) ou à la position zéro (85, 185).

2. Dispositif d'injection selon la revendication 1,
**caractérisé en ce que** le ressort (82, 182) est contraint lors du réglage d'une dose d'injection.

3. Dispositif d'injection selon la revendication 1 ou 2,
**caractérisé en ce que** le ressort (82, 182) agit entre l'organe de dosage (16, 116) et le boîtier (2, 102).

4. Dispositif d'injection selon l'une des revendications 1 à 3,
**caractérisé en ce que** le dispositif d'enclenchement (26, 126), dans les positions intermédiaires (74) de l'organe de dosage (16, 116), n'exerce pas de force sur l'organe de dosage (16, 116).

5. Dispositif d'injection selon l'une des revendications 1 à 4,
**caractérisé en ce qu'**il est prévu au moins deux positions d'injection (73, 173), qui présentent dans le sens circonférentiel autour de l'axe longitudinal médian (50) un écartement angulaire (β, δ) d'au moins environ 30°.

6. Dispositif d'injection selon l'une des revendications 1 à 5,
**caractérisé en ce que** l'élément de commande (6, 106) est en plusieurs parties et comporte un bouton d'actionnement (8, 108) et un manchon de réglage (7, 107), le manchon de réglage (7, 107) étant relié de manière fixe à l'organe de dosage, le bouton d'actionnement (8, 108) étant relié par l'intermédiaire d'un organe d'entraînement (13, 113) à l'élément de positionnement (20, 120), et le bouton d'actionnement (8, 108), pour éjecter du récipient le liquide d'injection, étant déplacé dans le sens de l'axe longitudinal médian (50) dans le sens proximal du dispositif d'injection (1, 101).

7. Dispositif d'injection selon la revendication 6,
**caractérisé en ce que** le bouton d'actionnement (8, 108) est relié par l'intermédiaire d'un accouplement (14, 114) au manchon de réglage (7, 107), l'accouplement (14, 114) réalisant dans une première position, distale (71, 171) du bouton d'actionnement (8, 108) une liaison fixe en rotation entre l'organe d'entraînement (13, 113) et le manchon de réglage (7, 107), et autorisant dans une seconde position, proximale (72) du bouton d'actionnement (8, 108) une rotation du manchon de réglage (7, 107) par rapport à l'organe d'entraînement (13, 113).

8. Dispositif d'injection selon l'une des revendications 1 à 7,
**caractérisé en ce que** le mouvement de rotation de l'élément de positionnement (20, 120), lors du réglage de la dose d'injection, provoque par l'intermédiaire d'une première liaison filetée (25, 125) un mouvement axial dudit élément de positionnement (20, 120), l'élément de positionnement (20, 120) étant déplacé dans le sens de l'axe longitudinal médian (50) du dispositif d'injection (1, 101) sur une première course de réglage (a, d).

9. Dispositif d'injection selon la revendication 8,
**caractérisé en ce que** l'organe de dosage (116) est monté en rotation et non coulissant dans le sens de l'axe longitudinal médian (50), dans le boîtier (102).

10. Dispositif d'injection selon la revendication 8 ou 9,
**caractérisé en ce que** le dispositif d'injection (101) comporte un coulisseau (119) qui porte un filetage d'une deuxième liaison filetée (121), le mouvement de rotation du coulisseau (119) provoquant un mouvement dans le sens distal de l'axe longitudinal médian (50) sur une deuxième course de réglage (e) qui est au moins aussi grande que la première course de réglage (d), et **en ce que** le coulisseau (119) comporte un épaulement d'entraînement (162) qui coopère avec un épaulement d'entraînement (163) de l'élément de positionnement (120) et qui transmet un mouvement axial du coulisseau (119) dans le sens proximal à l'élément de positionnement (120).

11. Dispositif d'injection selon la revendication 10,
**caractérisé en ce que** l'organe de dosage (16) est relié par l'intermédiaire d'une deuxième liaison filetée (18) au boîtier (2), le mouvement de rotation de l'organe de dosage (16) provoquant un mouvement de l'organe de dosage (16) et de l'élément de commande (6) dans le sens de l'axe longitudinal médian (50) du dispositif d'injection (1) sur une deuxième course (b), ladite deuxième course de réglage (b) étant plus grande que la première course (a).

12. Dispositif d'injection selon la revendication 11,
**caractérisé en ce que** le dispositif d'injection (1) comporte un coulisseau (19) qui porte un filetage d'une troisième liaison filetée (21), le mouvement de rotation du coulisseau (19) provoquant un mouvement dans le sens distal de l'axe longitudinal médian (50) sur une troisième course de réglage (c) qui est au moins aussi grande que la première course de réglage (a), et **en ce que** le coulisseau (19) comporte un épaulement d'entraînement (62) qui coopère avec un épaulement d'entraînement (63) de l'élément de positionnement (20) et qui transmet un mouvement axial du coulisseau (19) dans le sens proximal à l'élément de positionnement (20).
